(19)

Europäisches
Patentamt
European
Patent Office
Office européen
des brevets

(11) **EP 4 491 738 A1**

(12) **EUROPEAN PATENT APPLICATION**
published in accordance with Art. 153(4) EPC

(43) Date of publication:
**15.01.2025 Bulletin 2025/03**

(21) Application number: **23766432.1**

(22) Date of filing: **13.02.2023**

(51) International Patent Classification (IPC):
*C12Q 1/06* (2006.01)          *C12M 1/00* (2006.01)
*C12M 1/34* (2006.01)

(52) Cooperative Patent Classification (CPC):
**C12M 1/00; C12M 1/34; C12Q 1/06**

(86) International application number:
**PCT/JP2023/004809**

(87) International publication number:
**WO 2023/171252 (14.09.2023 Gazette 2023/37)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **08.03.2022 JP 2022035507**

(71) Applicant: **CANON KABUSHIKI KAISHA
Tokyo 146-8501 (JP)**

(72) Inventor: **HIGUCHI, Akira
Kyoto-shi, Kyoto 613-0916 (JP)**

(74) Representative: **TBK
Bavariaring 4-6
80336 München (DE)**

(54) **CULTURE DEVICE, CELL DENSITY MEASUREMENT DEVICE, AND CELL DENSITY MEASUREMENT METHOD**

(57)    Provided is a culture apparatus including: a culture vessel configured to accommodate a culture solution for culturing cells; a light source configured to emit light toward the culture solution; a first optical sensor configured to measure an intensity of light exiting from the culture solution; a transmitted light intensity ratio measurement unit configured to measure a transmitted light intensity ratio which is a ratio of an intensity of transmitted light of the culture solution during cell culture and in a state in which the cells are not suspended to an intensity of transmitted light of an unused culture solution; and a cell density calculation unit configured to calculate a cell density of the culture solution. The cell density calculation unit is configured to, when the cell density calculation unit calculates the cell density at a certain timing during the cell culture: correct the intensity of the light exiting from the culture solution in a state in which the cells are suspended and being measured by the first optical sensor at the certain timing through use of the transmitted light intensity ratio measured at the certain timing. Further, the cell density calculation unit is configured to calculate the cell density at the certain timing based on the corrected intensity of the light and an equation of a calibration curve indicating a relationship between a cell density of a culture solution and an intensity of light exiting from the culture solution, the calibration curve being created in advance under a condition in which the culture solution is in an unused state.

FIG. 1

EP 4 491 738 A1

## Description

[Technical Field]

The present disclosure relates to a culture apparatus for culturing cells, and a cell density measurement device and a cell density measurement method for measuring a cell density of a culture solution.

[Background Art]

Hitherto, in expansion culture of cells, for example, cells reactivated from a cell bank are grown from the number of cells on the order of from $10^4$ to $10^6$ until a large quantity of cells on the order of from $10^9$ to $10^{10}$ are obtained. The basis of cell growth is to culture cells while maintaining a culture state, in particular, activity of cells, and one index thereof is an appropriate cell density range. For example, when expansion culture is performed with $10^5$ to $10^7$ cells/mL, although it depends on a cell type and culture environment, the culture is started at a small cell density within this range, and cells are grown until an allowable cell density is reached. However, when a very large number of cells are required, there is a limit in one culture, and hence subculture is required to be repeated while the amount of the culture solution is increased. For example, cells are cultured in a petri dish through use of several milliliters of culture solution. When the cell density in the petri dish reaches a predetermined density, the cells in the petri dish are divided and seeded into a plurality of petri dishes and then cultured. This operation is repeated until the amount of the culture solution becomes several tens of milliliters in total. Thus, the cells can be transferred to a culture vessel, for example, a flask, which has a capacity larger than that of the petri dish. After transfer to a large-capacity culture vessel is performed several times as described above, the cells are finally transferred to a culture bag that can accommodate a large amount of culture solution, such as a bioreactor as described in Patent Literature 1. In this culture bag, for example, culture is performed until the number of the cells reaches a required number through use of fifty liters of culture solution.

[Citation List]

[Patent Literature]

PTL 1: Japanese Patent Application Laid-Open No. 2014-507959

[Summary of Invention]

[Technical Problem]

Incidentally, an important index for maximizing the cell quality in expansion culture is the cell density. In a cell culture process, in order to perform culture in an appropriate cell density range, it is required to regularly measure the cell density. As a matter of course, manual cell counting and cell density calculation using a hemocytometer cannot be frequently performed. In particular, in a fed-batch culture method, culture is performed while adding a culture solution in a range with the set cell density, and hence there is a possibility that the addition amount of the culture solution cannot be appropriately controlled at the timing at which the cell density has reached the predetermined density. Further, as an index for grasping the cell activity, a change over time of the cell density, that is, a change in growth speed of the cells may be captured so that an appropriate feedback is given to the cell culture process, such as changing the cell density to a predetermined amount. The cell activity changes from moment to moment even in the same cell line depending on the influence of the culture environment or the like. Thus, in order to adjust a collection timing of cells due to a change in culture process in accordance therewith or to determine interruption or the like of the cell culture (when recovery of the cell activity cannot be expected), it is required to frequently grasp the change in cell density. In this manner, it is required to minimize losses in culture period of several tens of days and expensive culture medium. However, when the operation is manually performed as in the related art, there is a possibility that those appropriate timings are missed.

In view of the above, the present disclosure has an object to achieve frequent execution of measurement of a cell density with a stable accuracy during cell culture.

[Solution to Problem]

In order to achieve the above-mentioned technical object, according to one aspect of the present disclosure, there is provided a culture apparatus including: a culture vessel configured to accommodate a culture solution for culturing cells; a light source configured to emit light toward the culture solution; a first optical sensor configured to measure an intensity of light exiting from the culture solution; a transmitted light intensity ratio measurement unit configured to measure a transmitted light intensity ratio which is a ratio of an intensity of transmitted light of the culture solution during cell culture and in a state in which the cells are not suspended to an intensity of transmitted light of an unused culture solution; and a cell density calculation unit configured to calculate a cell density of the culture solution. The cell density calculation unit is configured to, when the cell density calculation unit calculates the cell density at a certain timing during the cell culture: correct the intensity of the light exiting from the culture solution in a state in which the cells are suspended and being measured by the first optical sensor at the certain timing through use of the transmitted light intensity ratio measured by the transmitted light intensity ratio measurement unit at the certain timing; and calculate the cell density at the certain timing based on the corrected intensity of the light and an equation of a calibration curve indicating a relationship between a cell density of a culture solution and an intensity of light exiting from the culture solution, the calibration curve being created in advance under a condition in which the culture solution is in an unused state.

Further, according to another aspect of the present disclosure, there is provided a cell density measurement device including: a light source configured to emit light toward the culture solution; an optical sensor configured to measure an intensity of light exiting from the culture solution; a transmitted light intensity ratio measurement unit configured to measure a transmitted light intensity ratio which is a ratio of an intensity of transmitted light of the culture solution during cell culture and in a state in which the cells are not suspended to an intensity of transmitted light of an unused culture solution; and a cell density calculation unit configured to calculate a cell density of the culture solution. The cell density calculation unit is configured to, when the cell density calculation unit calculates the cell density at a certain timing during the cell culture: correct the intensity of the light exiting from the culture solution in a state in which the cells are suspended and being measured by the optical sensor at the certain timing through use of the transmitted light intensity ratio measured by the transmitted light intensity ratio measurement unit at the certain timing; and calculate the cell density at the certain timing based on the corrected intensity of the light and an equation of a calibration curve indicating a relationship between a cell density of a culture solution and an intensity of light exiting from the culture solution, the calibration curve being created in advance under a condition in which the culture solution is in an unused state.

Further, according to still another aspect of the present disclosure, there is provided a cell density measurement method for measuring a cell density of a culture solution during cell culture, the cell density measurement method including: emitting, by a light source, light toward the culture solution; measuring, by an optical sensor, an intensity of light exiting from the culture solution; measuring a transmitted light intensity ratio which is a ratio of an intensity of transmitted light of the culture solution during the cell culture and in a state in which cells are not suspended to an intensity of transmitted light of an unused culture solution; and when calculating the cell density at a certain timing during the cell culture, correcting the intensity of the light exiting from the culture solution in a state in which the cells are suspended and being measured by the optical sensor at the certain timing through use of the transmitted light intensity ratio measured at the certain timing; and calculating the cell density at the certain timing based on the corrected intensity of the light and an equation of a calibration curve indicating a relationship between a cell density of a culture solution and an intensity of light exiting from the culture solution, the calibration curve being created in advance under a condition in which the culture solution is in an unused state.

[Advantageous Effects of Invention]

According to the present disclosure, frequent execution of measurement of the cell density with a stable accuracy during the cell culture can be achieved.

[Brief Description of Drawings]

[FIG. 1]

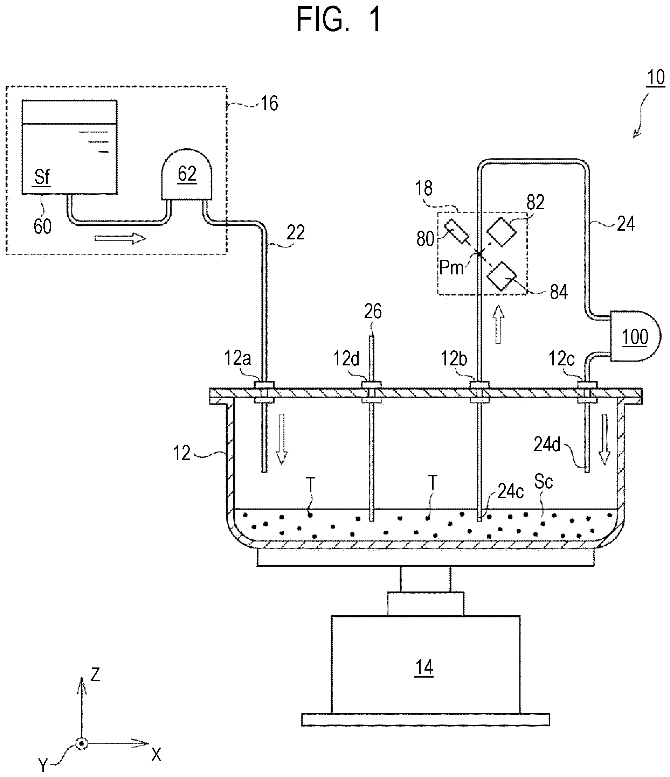

FIG. 1 is a schematic configuration view for illustrating a configuration of a culture apparatus according to a first embodiment of the present disclosure.
[FIG. 2]

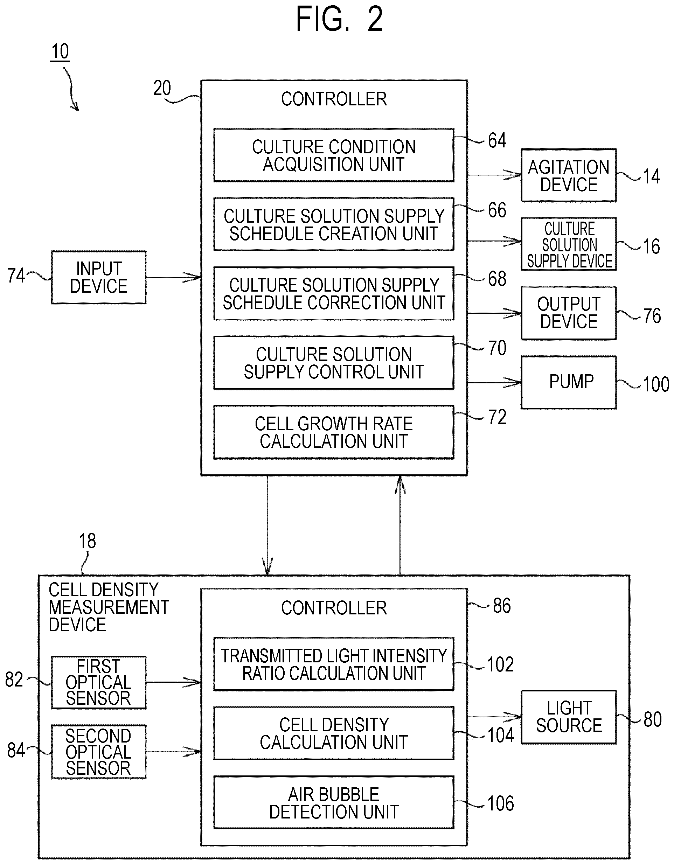

FIG. 2 is a block diagram for illustrating a control system of the culture apparatus.
[FIG. 3]

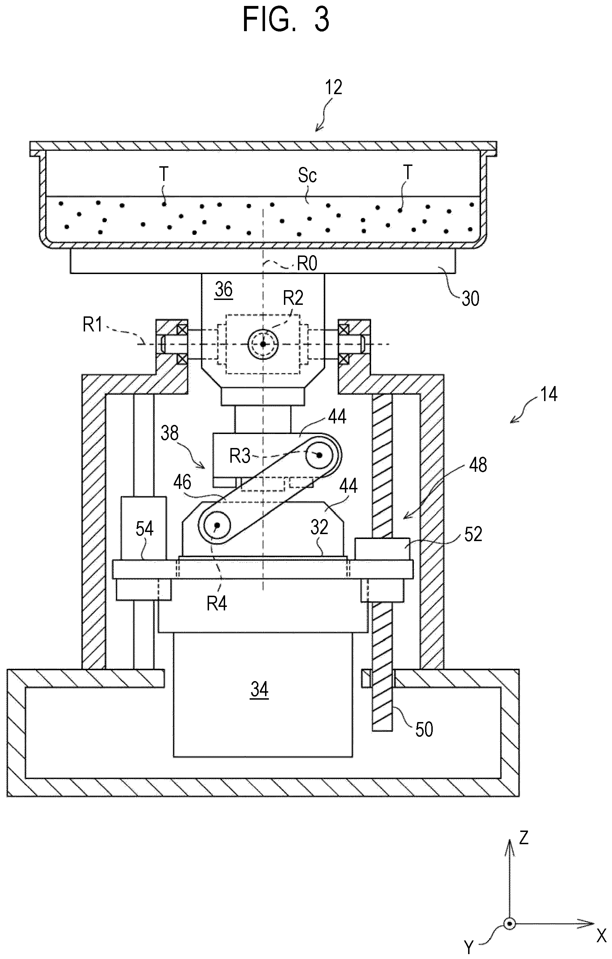

FIG. 3 is a schematic partial sectional view of an agitation device in the culture apparatus.
[FIG. 4]

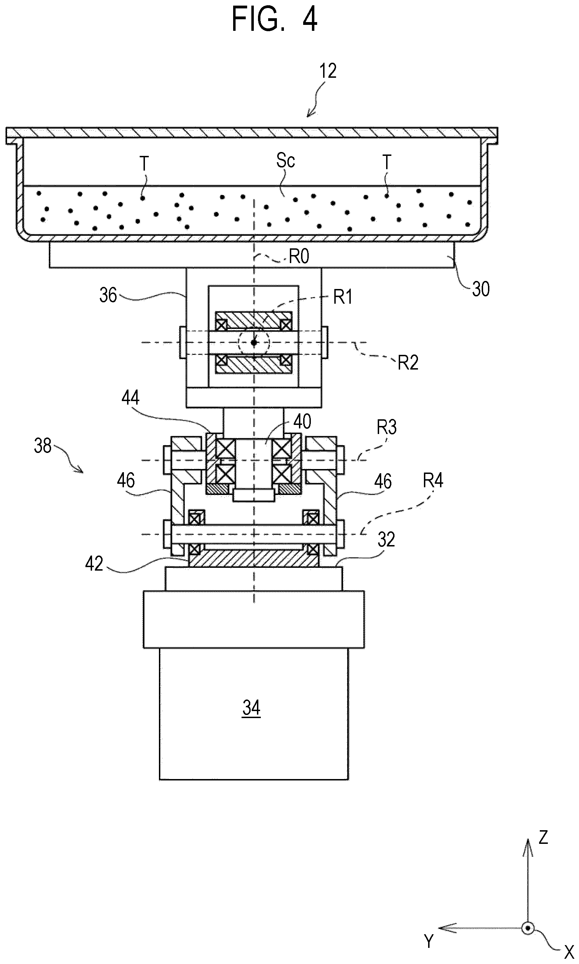

FIG. 4 is a schematic partial sectional view of a part of the agitation device as viewed from a different direction.
[FIG. 5]

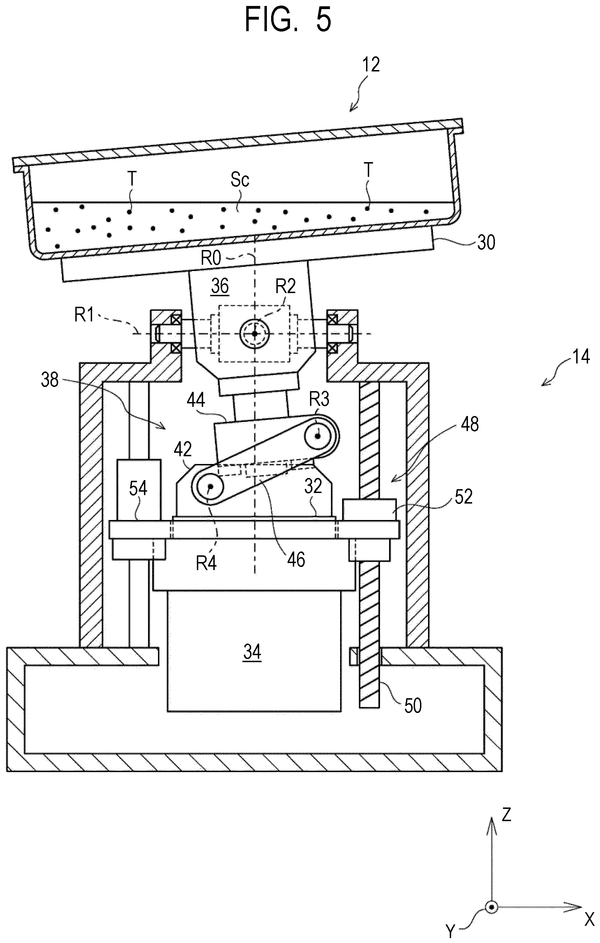

FIG. 5 is a schematic partial sectional view of the agitation device in a state in which a stage is inclined.
[FIG. 6]

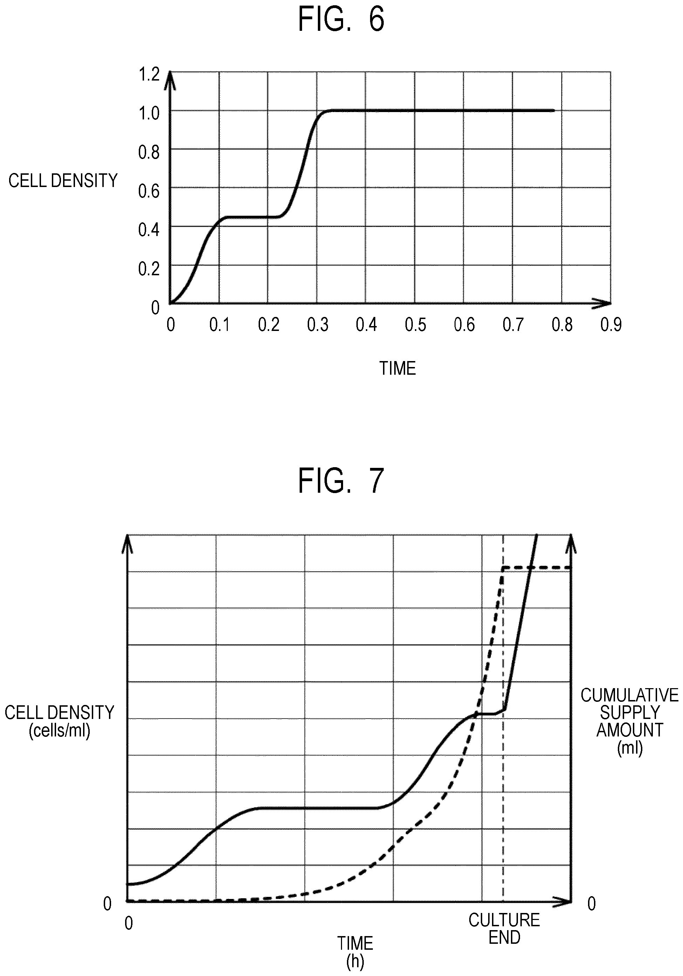

FIG. 6 is a graph for showing an example of a cell density profile.
[FIG. 7]
FIG. 7 is a graph for showing a cumulative culture solution supply amount with respect to a change over time of a cell density.
[FIG. 8]

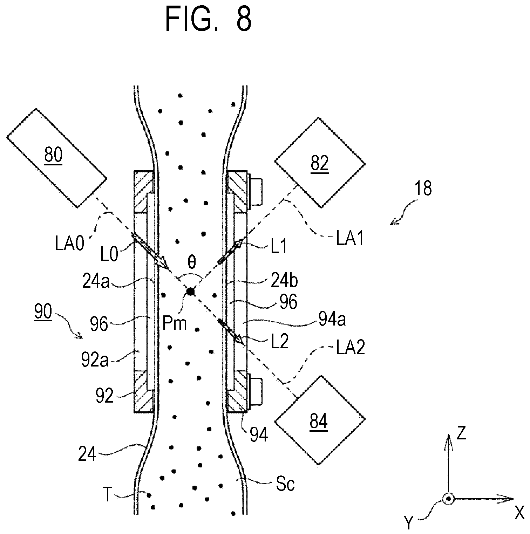

FIG. 8 is a schematic side view of a cell density measurement device.
[FIG. 9]

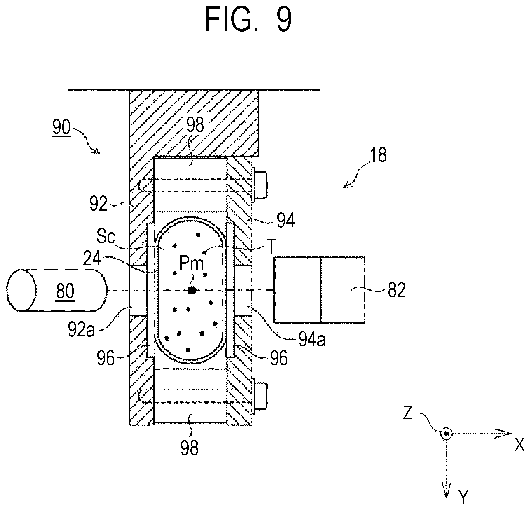

FIG. 9 is a schematic top view of the cell density measurement device.
[FIG. 10]

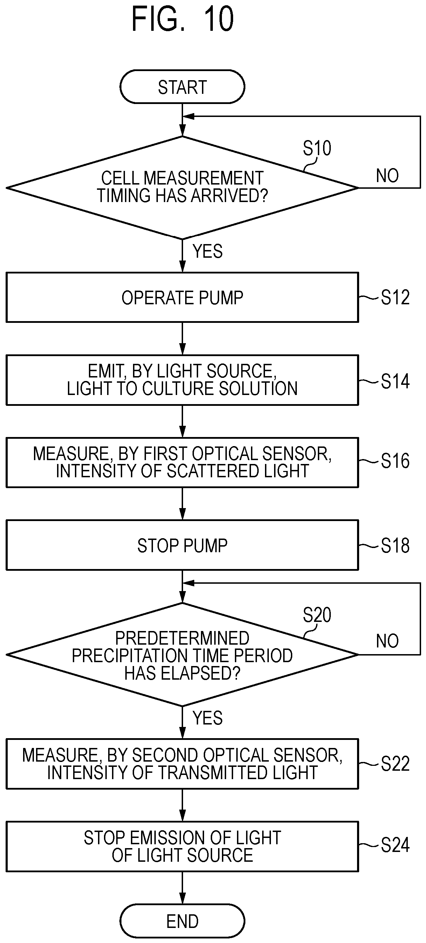

FIG. 10 is a flow chart for illustrating an example of a flow of measurement of an intensity of each of scattered light and transmitted light exiting from a culture solution.
[FIG. 11A]

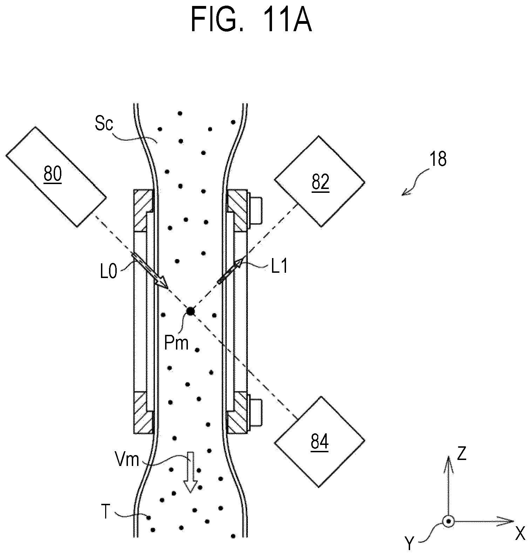

FIG. 11A is a view for illustrating the culture solution in a state in which cells are dispersed and when a first optical sensor measures the intensity of the scattered light.
[FIG. 11B]

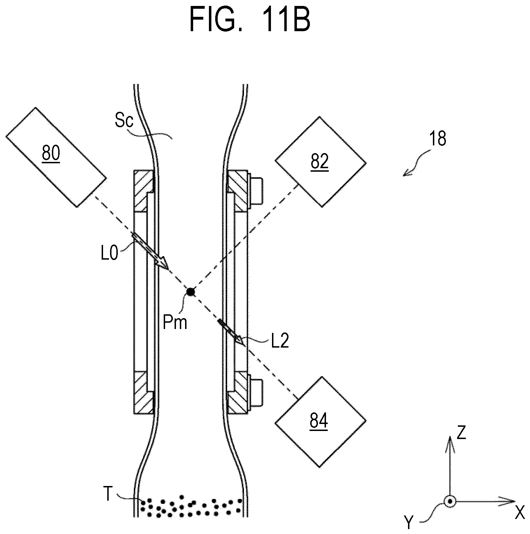

FIG. 11B is a view for illustrating the culture solution in a state in which the cells are precipitated and when a second optical sensor measures the intensity of the transmitted light.
[FIG. 12]

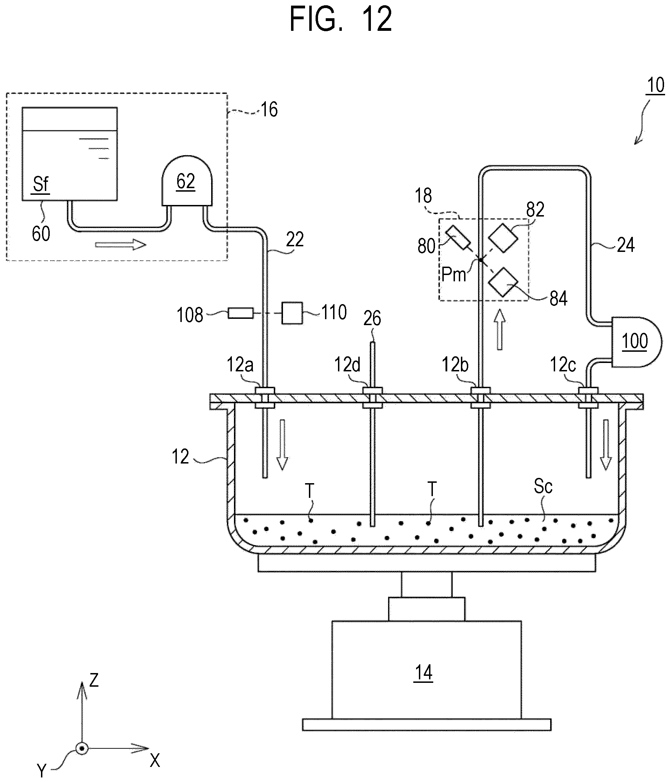

FIG. 12 is view for illustrating a modification mode of the culture apparatus according to the first embodiment.

[FIG. 13]

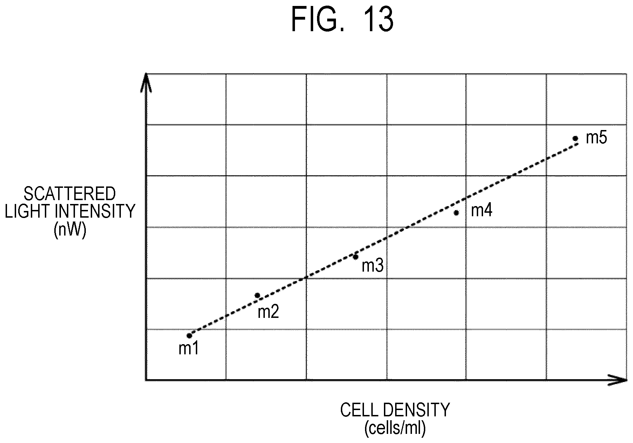

FIG. 13 is a graph for showing an example of a calibration curve indicating a relationship between a cell density of a culture solution and an intensity of scattered light exiting from this culture solution.

[FIG. 14A]

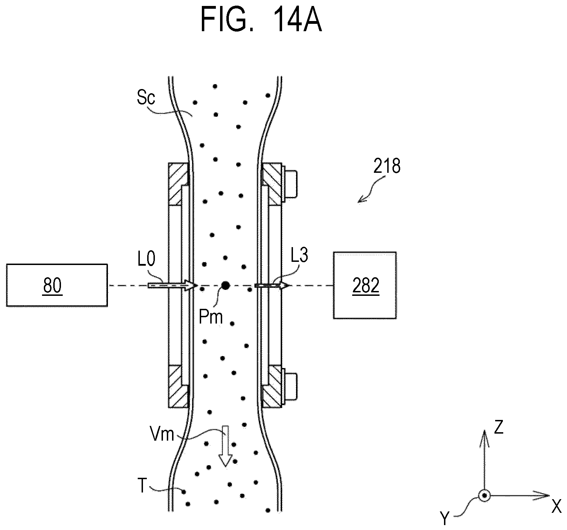

FIG. 14A is a view for illustrating a culture solution in a state in which cells are dispersed and when an optical sensor measures an intensity of transmitted light in a culture apparatus according to a second embodiment.

[FIG. 14B]

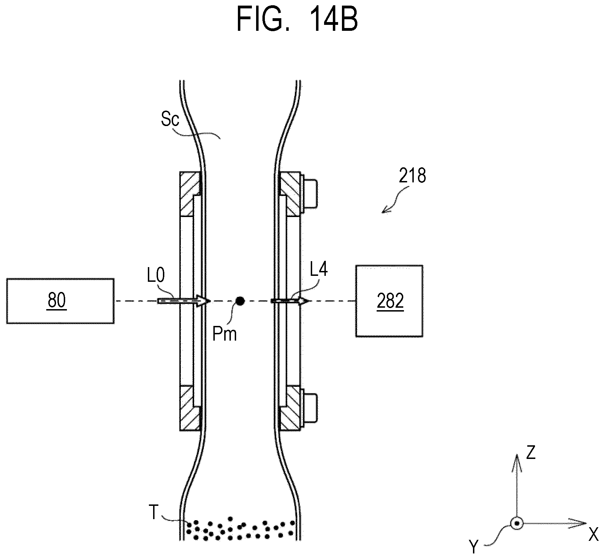

FIG. 14B is a view for illustrating the culture solution in a state in which the cells are precipitated and when the optical sensor measures an intensity of transmitted light in the culture apparatus according to the second embodiment.

[Description of Embodiments]

The culture apparatus according to one aspect of the present disclosure includes: a culture vessel configured to accommodate a culture solution for culturing cells; a light source configured to emit light toward the culture solution; a first optical sensor configured to measure an intensity of light exiting from the culture solution; a transmitted light intensity ratio measurement unit configured to measure a transmitted light intensity ratio which is a ratio of an intensity of transmitted light of the culture solution during cell culture and in a state in which the cells are not suspended to an intensity of transmitted light of an unused culture solution; and a cell density calculation unit configured to calculate a cell density of the culture solution. The cell density calculation unit is configured to, when the cell density calculation unit calculates the cell density at a certain timing during the cell culture: correct the intensity of the light exiting from the culture solution in a state in which the cells are suspended and being measured by the first optical sensor at the certain timing through use of the transmitted light intensity ratio measured by the transmitted light intensity ratio measurement unit at the certain timing; and calculate the cell density at the certain timing based on the corrected intensity of the light and an equation of a calibration curve indicating a relationship between a cell density of a culture solution and an intensity of light exiting from the culture solution, the calibration curve being created in advance under a condition in which the culture solution is in an unused state.

According to this aspect, during the cell culture, the measurement of the cell density can be frequently executed with a stable accuracy.

For example, the first optical sensor may be configured to measure an intensity of scattered light scattered from the cells suspended in the culture solution into which the light from the light source has entered. In this case, the transmitted light intensity ratio measurement unit includes: a second optical sensor opposed to the light source across the culture solution, the second optical sensor being configured to measure an intensity of transmitted light transmitted through the culture solution in the state in which the cells are not suspended; and a transmitted light intensity ratio calculation unit configured to calculate the transmitted light intensity ratio of the culture solution based on the intensity of the transmitted light measured by the second optical sensor. The equation of the calibration curve is an equation indicating a relationship between a cell density of a culture solution and an intensity of scattered light exiting from the culture solution, the equation being created in advance under a condition in which the culture solution is in an unused state. The cell density calculation unit is configured to correct the intensity of the scattered light by dividing the intensity of the scattered light by the transmitted light intensity ratio calculated by the transmitted light intensity ratio calculation unit.

For example, the transmitted light intensity ratio calculation unit may be configured to calculate the transmitted light intensity ratio at the certain timing by dividing the intensity of the transmitted light measured by the second optical sensor at the certain timing by the intensity of the transmitted light measured by the second optical sensor after being transmitted through a culture solution in a state in which the cells are not suspended and immediately before culture start.

For example, the culture apparatus may further include: a pipe for supply through which the unused culture solution is supplied to the culture vessel; a second light source configured to emit light to the culture solution in the pipe for supply; and a third optical sensor opposed to the second light source across the culture solution in the pipe for supply, the third optical sensor being configured to measure an intensity of transmitted light transmitted through the culture solution in the pipe for supply. In this case, the transmitted light intensity ratio calculation unit is configured to calculate the transmitted light intensity ratio at the certain timing by dividing the intensity of the transmitted light measured by the second optical sensor at the certain timing by the intensity of the transmitted light measured by the third optical sensor.

For example, the culture apparatus may further include: a pipe for measurement connected to the culture vessel, the pipe for measurement including a part extending in a direction including at least a vertical direction component, the part passing between the light source and the first optical sensor and between the light source and the second optical sensor; and a pump configured to supply the culture solution in the culture vessel into the pipe for measurement. In this case, the light source is configured to emit light toward a measurement point in the pipe for measurement. The second optical sensor is configured to measure the intensity of the transmitted light transmitted through the culture solution present at the measurement point, under a state in which the cells precipitate at a position in the pipe for measurement below the measurement point due to stop of the pump. The first optical sensor is configured to measure the intensity of the scattered light scattered from the cells suspended in the culture solution present at the measurement position, under a state in which the culture solution flows through the pipe for measurement due to an operation of the pump.

For example, the pipe for measurement may be a tube that is transparent and has flexibility. In this case, the culture apparatus further includes a clamp mechanism configured to sandwich the tube to form an incident surface and an exit surface in the tube, the incident surface and the exit surface being formed in parallel to each other and each having a planar shape. The light source is configured to emit light toward the incident surface of the tube, and the first optical sensor and the second optical sensor are configured to receive scattered light and transmitted light, respectively, exiting from the exit surface of the tube.

For example, the culture apparatus may further include an air bubble detection unit configured to determine that an air bubble is present inside of the pipe for measurement when the intensity of the scattered light measured by the first optical sensor is larger than the intensity of the scattered light measured at a timing immediately before to exceed a predetermined threshold value.

In place thereof or in addition thereto, the air bubble detection unit may be configured to determine the presence of the air bubble in a case in which, when the intensity of the transmitted light measured by the second optical sensor is viewed in time-series data, a standard deviation or a coefficient of variation of the data is large to exceed a predetermined threshold value.

For example, the pump may be configured to perform a forward rotation operation and a reverse rotation operation, and the pipe for measurement may be connected to the culture vessel so that an inlet end thereof enters the culture solution and an outlet end thereof is separated away from a liquid surface of the culture solution. In this case, the pump is configured to execute, when the air bubble detection unit determines that the air bubble is present in the pipe for measurement, the reverse rotation operation to introduce air into the pipe for measurement via the outlet end so that the culture solution in the pipe for measurement is replaced by the air, and execute the forward rotation operation after the replacement is completed to supply the culture solution into the pipe for measurement.

For example, the pump may be configured to perform a forward rotation operation and a reverse rotation operation. In this case, the pump is configured to alternately repeat, when the air bubble detection unit determines that the air bubble is present in the pipe for measurement, the forward rotation operation and the reverse rotation operation so that a pulsating flow of the culture solution is caused in the pipe for measurement.

For example, the pump may be configured to supply, when the air bubble detection unit determines that the air bubble is present in the pipe for measurement, the culture solution into the pipe for measurement at a flow velocity slower than a flow velocity used when the first optical sensor measures the intensity of the scattered light.

For example, it is preferred that, when an optical axis of the light source and an optical axis of the second optical sensor match each other, and an optical axis of the first optical sensor intersects with the optical axis of the light source, a distance from an intersection between the optical axis of the light source and the optical axis of the first optical sensor to a light receiving surface of the second optical sensor be a distance of $\pm20\%$ or less with respect to a distance from the intersection to a light receiving surface of the first optical sensor.

For example, it is preferred that an angle formed between the optical axis of the light source and the optical axis of the first optical sensor be an angle falling within a range of from 0 degrees to 90 degrees.

For example, the angle formed between the optical axis of the light source and the optical axis of the first optical sensor may be adjustable.

For example, an optical element may be arranged between the second optical sensor and the culture solution, the optical element being configured to change the intensity of the transmitted light entering the second optical sensor.

For example, the first optical sensor may be opposed to the light source across the culture solution, and may be configured to measure an intensity of transmitted light transmitted through the culture solution in the state in which the cells are suspended. In this case, the transmitted light intensity ratio measurement unit includes a transmitted light intensity ratio calculation unit configured to calculate a transmitted light intensity ratio of the culture solution based on the intensity of the transmitted light transmitted through the culture solution in the state in which the cells are not suspended and measured by the first optical sensor. The equation of the calibration curve is an equation indicating a relationship between a cell density of a culture solution and an intensity of transmitted light exiting from the culture solution, the equation being created in advance under a condition in which the culture solution is in an unused state. The cell density calculation unit is configured to correct the intensity of the transmitted light by dividing the intensity of the transmitted light by the transmitted light intensity ratio calculated by the transmitted light intensity ratio calculation unit.

For example, the culture apparatus may further include a culture solution supply control unit configured to control a supply rate of the unused culture solution to be supplied to the culture vessel. The culture solution supply control unit is configured to control the supply rate of the culture solution based on the cell density calculated by the cell density calculation unit.

For example, the culture apparatus may further include a cell growth rate calculation unit configured to calculate a cell growth rate based on the cell density calculated by the cell density calculation unit at each of two successive timings. The culture solution supply control unit is configured to control the supply rate of the culture solution based on the cell growth rate calculated by the cell growth rate calculation unit.

For example, the culture apparatus may further include: a culture solution supply schedule creation unit configured to create a culture solution supply schedule based on a doubling time of the cells, a target cell number, and a cell density profile indicating a suitable change over time of the cell density; and a culture solution supply schedule correction unit configured to correct the culture solution supply schedule based on the cell density calculated by the cell density calculation unit. The culture solution supply control unit is configured to control the supply rate of the culture solution in accordance with the culture solution supply schedule.

Further, a cell density measurement device according to another aspect of the present disclosure includes: a light source configured to emit light toward the culture solution; an optical sensor configured to measure an intensity of light exiting from the culture solution; a transmitted light intensity ratio measurement unit configured to measure a transmitted light intensity ratio which is a ratio of an intensity of transmitted light of the culture solution during cell culture and in a state in which the cells are not suspended to an intensity of transmitted light of an unused culture solution; and a cell density calculation unit configured to calculate a cell density of the culture solution. The cell density calculation unit is configured to, when the cell density calculation unit calculates the cell density at a certain timing during the cell culture: correct the intensity of the light exiting from the culture solution in a state in which the cells are suspended and being measured by the optical sensor at the certain timing through use of the transmitted light intensity ratio measured by the transmitted light intensity ratio measurement unit at the certain timing; and calculate the cell density at the certain timing based on the corrected intensity of the light and an equation of a calibration curve indicating a relationship between a cell density of a culture solution and an intensity of light exiting from the culture solution, the calibration curve being created in advance under a condition in which the culture solution is in an unused state.

According to this aspect, during the cell culture, the measurement of the cell density can be frequently executed with a stable accuracy.

Further, a cell density measurement method according to still another aspect of the present disclosure is a cell density measurement method for measuring a cell density of a culture solution during cell culture, the cell density measurement method including: emitting, by a light source, light toward the culture solution; measuring, by an optical sensor, an intensity of light exiting from the culture solution; measuring a transmitted light intensity ratio which is a ratio of an intensity of transmitted light of the culture solution during the cell culture and in a state in which cells are not suspended to an intensity of transmitted light of an unused culture solution; and when calculating the cell density at a certain timing during the cell culture, correcting the intensity of the light exiting from the culture solution in a state in which the cells are suspended and being measured by the optical sensor at the certain timing through use of the transmitted light intensity ratio measured at the certain timing; and calculating the cell density at the certain timing based on the corrected intensity of the light and an equation of a calibration curve indicating a relationship between a cell density of a culture solution and an intensity of light exiting from the culture solution, the calibration curve being created in advance under a condition in which the culture solution is in an unused state.

According to this aspect, during the cell culture, the measurement of the cell density can be frequently executed with a stable accuracy.

Hereinbelow, with reference to the drawings, description is given of embodiments of the present disclosure.

(First Embodiment)

FIG. 1 is a schematic view for illustrating a configuration of a culture apparatus according to a first embodiment of the present disclosure. Further, FIG. 2 is a block diagram for illustrating a control system of the culture apparatus. An X-Y-Z orthogonal coordinate system shown in the drawings is for facilitating understanding of embodiments of the present disclosure, and does not limit the embodiments of the present disclosure. A Z axis direction indicates the vertical direction, and an X axis direction and a Y axis direction indicate the horizontal direction.

As illustrated in FIG. 1 and FIG. 2, in the case of the first embodiment, a culture apparatus 10 includes a culture vessel 12 for accommodating a culture solution Sc including cells T, and an agitation device 14 for rocking the culture vessel 12 in order to agitate the culture solution Sc in the culture vessel 12, to thereby agitate the culture solution Sc therein. The culture apparatus 10 further includes a culture solution supply device 16 for supplying an unused culture solution Sf (new culture solution including no cells T) to the culture vessel 12. The culture apparatus 10 further includes a cell density measurement device 18 for measuring a cell density of the cells T in the culture vessel 12, and a controller 20 for controlling the agitation device 14 and the culture solution supply device 16.

The culture vessel 12 is a vessel for accommodating the culture solution Cs including the cells T, and includes a plurality of ports 12a, 12b, 12c, and 12d. A pipe 22 for supply, such as a tube, is inserted into the port 12a. The unused culture solution Sf to be supplied into the culture vessel 12 from the culture solution supply device 16 flows through the pipe 22 for supply. Further, although details are described later, a pipe 24 for measurement, such as a tube, is inserted into the ports 12b and 12c. The culture solution Sc whose cell density is to be measured by the cell density measurement device 18 flows through the pipe 24 for measurement. Further, a pipe 26 for sampling, such as a tube, is inserted into the port 12d in order to take a sample of the culture solution Sc.

The agitation device 14 is configured to support the culture vessel 12 and is controlled by the controller 20 to change a posture (inclination) of the culture vessel 12.

FIG. 3 is a schematic partial sectional view of the agitation device in the culture apparatus. Further, FIG. 4 is a schematic partial sectional view of a part of the agitation device as viewed from a different direction. Moreover, FIG. 5 is a schematic partial sectional view of the agitation device in a state in which a stage is inclined.

As illustrated in FIG. 3 and FIG. 4, the agitation device 14 includes a stage 30 on which the culture vessel 12 is to be placed, and a rotary actuator 34 including a rotary table 32 which rotates about a rotation center axis R0 extending in the vertical direction (Z axis direction).

The stage 30 and the rotary actuator 34 are drivingly coupled to each other via a rocking head 36 and a tilting mechanism 38.

The rocking head 36 is provided in the agitation device 14 so as to support the stage 30 and to be rockable about a rocking axis R1 extending in the horizontal direction (X axis direction) and a rocking axis R2 extending in the horizontal direction (Y axis direction) and being orthogonal to the rocking axis R11. Further, the rocking head 36 includes, in a lower portion thereof, a coupling shaft 40 to be drivingly coupled to the rotary actuator 34 via the tilting mechanism 38. When the stage 30 takes a horizontal posture, the coupling shaft 40 of the rocking head 36 extends in the vertical direction (Z axis direction).

The tilting mechanism 38 is a link mechanism for inclining the stage 30 via the rocking head 36, that is, inclining the culture vessel 12 on the stage 30 with respect to the horizontal direction. Thus, the tilting mechanism 38 includes a base portion 42, a rocking head coupling portion 44 coupled to the rocking head 36, and link arms 46 for coupling the base portion 42 and the rocking head coupling portion 44 to each other.

The base portion 42 of the tilting mechanism 38 is mounted to the rotary table 32 of the rotary actuator 34. Thus, when the rotary actuator 34 is driven, the base portion 42 rotates together with the rotary table 32 about the rotation center axis R0.

The rocking head coupling portion 44 of the tilting mechanism 38 is inserted over the coupling shaft 40 of the rocking head 36 so as to be slidable through intermediation of, for example, a bearing or the like.

The link arms 46 of the tilting mechanism 38 are configured to couple the base portion 42 and the rocking head coupling portion 44 to each other. Specifically, each of the link arms 46 includes one end turnably fixed to the rocking head coupling portion 44, and another end turnably fixed to the base portion 42. A turning axis R3 at the one end of each of the link arms 46 and a rotation axis R4 at the another end thereof each extend in the horizontal direction, and are parallel to each other.

The rotary actuator 34 having the base portion 42 of the tilting mechanism 38 mounted thereon is raised and lowered in the vertical direction (Z axis direction) by a ball screw mechanism 48.

The ball screw mechanism 48 includes a screw shaft 50 extending in the vertical direction (Z axis direction), a nut 52 engaged with the screw shaft 50, and a motor (not shown) for rotating the screw shaft 50. The nut 52 is mounted to a raising/lowering bracket 54. The rotary actuator 34 is mounted to this raising/lowering bracket 54.

When the ball screw mechanism 48 is driven, the rotary actuator 34 is raised and lowered together with the raising/lowering bracket 54 via the nut 52. For example, as illustrated in FIG. 5, when the rotary actuator 34 is raised by the ball screw mechanism 48, the stage 30 is inclined via the tilting mechanism 38. Specifically, the base portion 42 of the tilting mechanism 38 mounted to the rotary actuator 34 is raised so that the link arms 46 push the rocking head coupling portion 44. Thus, the rocking head 36 rotates together with the rocking head coupling portion 44 about at least one of the rocking axis R1 or the rocking axis R2 (rocking axis R2 in FIG. 5). In this manner, the stage 30 is inclined, and the culture vessel 12 on this stage 30 is also inclined.

As illustrated in FIG. 5, when the rotary actuator 34 is driven under a state in which the stage 30 is inclined and thus the rotary table 32 is rotated, the tilting mechanism 38 rotates about the rotation center axis R0 so that an inclination direction of the stage 30 changes. As a result, the culture solution Sc in the culture vessel 12 is agitated and the cells in the culture solution Sc are cultured.

In such an agitation device 14, even when the rotary actuator 34 rotates the tilting mechanism 38, for example, one revolution, the stage 30 itself does not rotate, and only the inclination direction of the stage 30 is rotated one revolution instead. That is, a lowest part of the culture vessel 12 on the stage 30 is only sequentially changed to another part so as to circle around in top view (as viewed in Z axis direction). As a result, the culture solution Sc circles around to be agitated.

With such an agitation device 14, the expansion culture can be executed through use of only one culture vessel 12. Specifically, when agitating conditions (inclination angle of the stage 30, and rotation angle range and rotation speed of the rotary actuator 34) are changed depending on a liquid amount of the culture solution Sc in the culture vessel 12, culture solutions Sc in a small amount to a large amount can be agitated with agitating conditions suitable for cell growth.

Referring back to FIG. 1 and FIG. 2, the culture solution supply device 16 of the culture apparatus 10 includes a storage vessel 60 for storing the unused culture solution Sf, which is connected to the pipe 22 for supply, and a pump 62 for sending the culture solution Sf in the storage vessel 60 to the culture vessel 12 through the pipe 22 for supply. The pump 62 is, for example, a roller pump, and is controlled by the controller 20.

The cell density measurement device 18 of the culture apparatus 10 measures the cell density of the culture solution Sc in the culture vessel 12. Details of the cell density measurement device 18 are described later.

As illustrated in FIG. 2, the controller 20 of the culture apparatus 10 controls the agitation device 14 (rotary actuator 34 thereof and ball screw mechanism 48) and the culture solution supply device 16 (pump 62 thereof) to control the culture of the cells T. The controller 20 includes, for example, a processor, such as a CPU or an MPU, a storage device such as a memory for storing a program for causing the processor to execute various operations, and a circuit for electrically connecting the processor to an external device of the controller 20, such as the agitation device 14, the culture solution supply device 16, or the cell density measurement device 18.

As illustrated in FIG. 2, the controller 20 includes a culture condition acquisition unit 64 for acquiring culture conditions from outside, a culture solution supply schedule creation unit 66, a culture solution supply schedule correction unit 68, a culture solution supply control unit 70, and a cell growth rate calculation unit 72. For example, the processor operates in accordance with the program stored in the storage device so as to function as the culture condition acquisition unit 64, the culture solution supply schedule creation unit 66, the culture solution supply schedule correction unit 68, the culture solution supply control unit 70, or the cell growth rate calculation unit 72.

The culture condition acquisition unit 64 of the controller 20 acquires, from the outside, information (data) on a doubling time of the cells T, a target cell number, a cell density at culture start, and a cell density profile as the culture conditions required for cell culture. The information on the culture conditions is acquired from a user via an input device 74 and an output device 76. Examples of the input device 74 include a keyboard, a mouse, a touch screen, and a card reader for reading a memory card. Examples of the output device 76 include a display, a voice, and a touch screen.

FIG. 6 shows an example of the cell density profile.

As shown in FIG. 6, the cell density profile is information indicating a change over time of the cell density suitable for growth of the cells T, and varies depending on the type of the cells T. For example, the cell density profile is created based on a Gompertz curve or a logistic curve which is a growth curve. The cell density profile shown in FIG. 6 is normalized.

The culture solution supply schedule creation unit 66 of the controller 20 creates a supply schedule of the unused culture solution Sf to be supplied into the culture vessel 12 by the culture solution supply device 16, based on the doubling time, the target cell number, the cell density at culture start, and the cell density profile acquired via the culture condition acquisition unit 64.

FIG. 7 is a graph for showing a cumulative culture solution supply amount with respect to the change in cell density.

As shown in FIG. 7, the culture solution supply schedule creation unit 66 first calculates, based on the doubling time, the target cell number, the cell density at culture start, and the cell profile, a change over time (solid line) of the cell density until the target cell number is reached (until the culture is ended) and a change over time (broken line) of the cumulative supply amount of the culture solution Sf of the culture solution supply device 16 to be required for achieving this change in cell density. The change over time of the cumulative supply amount is, in other words, a change over time of the amount of the culture solution Sc in the culture vessel 12. The culture solution supply schedule creation unit 66 creates the culture solution supply schedule based on the calculated change over time of the cumulative supply amount. The culture solution supply schedule indicates an elapsed time from the start of the culture and the cumulative supply amount (expected cumulative supply amount) expected in this elapsed time.

The culture solution supply schedule correction unit 68 of the controller 20 corrects the culture solution supply schedule based on the cell density of the culture solution Sc in the culture vessel 12 measured by the cell density measurement device 18. During cell culture, the actual change in cell density may be different from the change in cell density (expected change in cell density) calculated by the culture solution supply schedule creation unit 66. When the difference between the actual cell density and the expected cell density at a certain timing exceeds a predetermined threshold value, the culture solution supply schedule correction unit 68 corrects the culture solution supply schedule after this certain timing. For example, when the actual cell density is higher than the expected cell density, the culture solution supply schedule correction unit 68 corrects the culture solution supply schedule so that the supply amount of the culture solution is increased.

The culture solution supply control unit 70 of the controller 20 controls the culture solution supply device 16 so as to control the supply rate of the unused culture solution Sf to be supplied to the culture vessel 12. In the case of the first embodiment, the culture solution supply control unit 70 controls the pump 62 of the culture solution supply device 16. Further, the culture solution supply control unit 70 controls the supply rate of the culture solution Sf based on the culture solution supply schedule created by the culture solution supply schedule creation unit 66 or corrected by the culture solution supply schedule correction unit 68.

The cell growth rate calculation unit 72 of the controller 20 calculates the cell growth rate based on the cell density measured by the cell density measurement device 18 at each of two successive timings or at non-successive timings having any interval. Specifically, the cell growth rate calculation unit 72 calculates the number of cells based on the measured cell density and the amount of the culture solution Sc in the culture vessel 12 at this measurement timing. The cell growth rate calculation unit 72 calculates the cell growth rate through use of the number of cells at each of the two successive timings and a time period between those two timings. When the cell growth rate calculated by the cell growth rate calculation unit 72 is different from the expected cell growth rate calculated from the doubling time and the cell density profile to exceed a predetermined threshold value, the culture solution supply control unit 70 controls the supply rate of the culture solution Sf based on the cell growth rate calculated by the cell growth rate calculation unit 72. In the case of a certain type of cell, the supply rate of the culture solution Sf is increased when the cell growth rate is reduced, and in the case of another type of cell, the supply rate of the culture solution Sf is decreased when the cell growth rate is reduced.

As described above, the control of the supply rate of the culture solution Sf is executed by the culture solution supply control unit 70 based on the cell density measured by the cell density measurement device 18. Now, details of the cell density measurement device 18 are described.

As illustrated in FIG. 1 and FIG. 2, in the case of the first embodiment, the cell density measurement device 18 includes a light source 80 for emitting light toward the culture solution Sc, a first optical sensor 82 and a second optical sensor 84 each for measuring light exiting from the culture solution Sc, and a controller 86.

FIG. 8 and FIG. 9 are a schematic side view and a schematic top view of the cell density measurement device, respectively.

As illustrated in FIG. 8 and FIG. 9, in the case of the first embodiment, the cell density measurement device 18 is provided on the pipe 24 for measurement. The cell density measurement device 18 measures the cell density of the culture solution Sc at a measurement point Pm in the pipe 24 for measurement.

The light source 80 is, for example, an LED, and emits light L0 at a certain intensity toward the culture solution Sf at the measurement point Pm in the pipe 24 for measurement. In the case of the first embodiment, an optical axis LA0 of the light source 80 intersects with an extending direction (Z axis direction) of the pipe 24 for measurement, that is, a flow direction of the culture solution Sf.

The first optical sensor 82 is, for example, a photodiode, and measures an intensity of light L1 exiting from the culture solution Sc in the pipe 24 for measurement. In the case of the first embodiment, the first optical sensor 82 measures an intensity of scattered light L1 scattered from the cells T suspended in the culture solution Sc at the measurement point Pm in the pipe 24 for measurement. The "suspended" state as used herein refers to a state in which the cells are dispersed in the culture solution so that the cell density becomes uniform in the culture solution.

In order to measure the intensity of the scattered light L1, in the case of the first embodiment, an optical axis LA1 of the first optical sensor 82 intersects with the optical axis LA0 of the light source 80 at an angle "θ". It is preferred that the angle "θ" fall within a range of from 0 degrees to 90 degrees. The reason therefor is because, when the angle "θ" exceeds 90 degrees, there is a possibility that light other than the scattered light, that is, transmitted light transmitted through the culture solution Sc may enter the first optical sensor 82.

The second optical sensor 84 is, for example, a photodiode, and, in the case of the first embodiment, measures an intensity of light L2 exiting from the pipe 24 for measurement. Further, the second optical sensor 84 measures an intensity of transmitted light L2 that has transmitted through the culture solution Sf and has passed through the measurement point Pm.

In order to measure the intensity of the transmitted light L2, in the case of the first embodiment, the second optical sensor 84 is opposed to the light source 80 across the culture solution Sf in the pipe 24 for measurement. An optical axis LA2 of the second optical sensor 84 matches the optical axis LA0 of the light source 80. In this manner, the transmitted light L2 that has transmitted through the culture solution Sf and has passed through the measurement point Pm enters the second optical sensor 84.

It is preferred that a distance from an intersection between the optical axis LA0 of the light source 80 and the optical axis LA1 of the first optical sensor 82 (that is, the measurement point Pm) to a light receiving surface of the second optical sensor 84 be a distance of ±20% or less with respect to a distance from the measurement point Pm to a light receiving surface of the first optical sensor 82. Thus, intensity measurement conditions of the first optical sensor 82 and the second optical sensor 84 can be set to be substantially equal to each other.

In the case of the first embodiment, although the reason is described later, a part of the pipe 24 for measurement passing between the light source 80 and the first optical sensor 82 and between the light source 80 and the second optical sensor 84 extends at least in the vertical direction (Z axis direction). This part of the pipe 24 for measurement is sandwiched by a clamp mechanism 90.

Specifically, in the case of the first embodiment, the pipe 24 for measurement is a transparent tube having flexibility. The clamp mechanism 90 includes a first clamp claw 92 and a second clamp claw 94 for clamping the pipe 24 for measurement. Each of the first clamp claw 92 and the second clamp claw 94 has a transparent glass plate 96 provided thereto. The pipe 24 for measurement is sandwiched between the first clamp claw 92 and the second clamp claw 94 via the transparent glass plates 96. Further, a spacer 98 is provided between the first clamp claw 92 and the second clamp claw 94. As a result, the pipe 24 for measurement has parallel planar parts 24a and 24b formed with an interval secured therebetween.

One planar part 24a of the pipe 24 for measurement functions as an incident surface which the light L0 from the light source 80 enters. That is, the light L0 from the light source 80 passes through a through hole 92a of the first clamp claw 92, is transmitted through the glass plate 96, and then enters the one planar part 24a of the pipe 24 for measurement. Further, another planar part 24b of the pipe 24 for measurement functions as an exit surface from which the scattered light L1 to be received by the first optical sensor 82 and the transmitted light L2 to be received by the second optical sensor 84 exit. That is, each of the scattered light L1 and the transmitted light L2 exiting from the culture solution Sc exits from the planar part 24b, is transmitted through the glass plate 96, and then enters a corresponding one of the first optical sensor 82 or the second optical sensor 84.

With such planar parts 24a and 24b of the pipe 24 for measurement, a large amount of light L0 from the light source 80 can enter the culture solution Sc in the pipe 24 for measurement, and large amounts of scattered light L1 and transmitted light L2 exiting from the culture solution Sc can exit to the outside of the pipe 24 for measurement.

Further, a width (size in the Y axis direction) of the through hole 94a of the clamp claw 94 is set to a size that prevents scattered light (stray light) exiting from parts other than the planar parts 24a and 24b of this sandwiched tube from being received by the first optical sensor 82. Thus, the first optical sensor 82 receives light whose scattered light component from the cells is maximized.

In order to supply the culture solution Sc in the culture vessel 12 into the pipe 24 for measurement, as illustrated in FIG. 1 and FIG. 2, the culture apparatus 10 includes a pump 100 such as a roller pump. When the cell density measurement device 18 measures the cell density of the culture solution Sc in the culture vessel 12, the pump 100 supplies the culture solution Sc into the pipe 24 for measurement.

The controller 86 of the cell density measurement device 18 includes, for example, a processor, such as a CPU or an MPU, a storage device such as a memory for storing a program for causing the processor to execute various operations, and a circuit for electrically connecting the processor to each of the light source 80, the first optical sensor 82, and the second optical sensor 84.

As illustrated in FIG. 2, the controller 86 of the cell density measurement device 18 includes a transmitted light intensity ratio calculation unit 102 for calculating a transmitted light intensity ratio of the culture solution Sc, a cell density calculation unit 104 for calculating the cell density of the culture solution Sc, and an air bubble detection unit 106 for detecting an air bubble in the pipe 24 for measurement. For example, the processor operates in accordance with the program stored in the storage device so as to function as the transmitted light intensity ratio calculation unit 102, the cell density calculation unit 104, or the air bubble detection unit 106.

The controller 20 of the culture apparatus 10 and the controller 86 of the cell density measurement device 18 may be integrated with each other. That is, the integrated controller includes one processor and one storage device.

The configuration of the cell density measurement device 18 has been described so far. Now, a method of measuring the cell density by the cell density measurement device 18 is described.

The measurement of the cell density includes intensity measurement of scattered light and transmitted light performed by the first optical sensor 82 and the second optical sensor 84, and cell density calculation based on the measured intensity of each of the scattered light and the transmitted light.

FIG. 10 is a flow chart for illustrating an example of a flow of measurement of the intensity of each of the scattered light and the transmitted light exiting from the culture solution.

As illustrated in FIG. 10, in Step S10, it is determined whether or not a cell density measurement timing has arrived. The cell density measurement timing is a timing to measure the cell density during culture of the cells T, and arrives at a predetermined time interval (for example, at an interval of one hour). Further, the cell density measurement timing arrives when the user gives an instruction to measure the cell density via the input device 74. When the cell density measurement timing has arrived, the process proceeds to next Step S 12.

When the cell density measurement timing has arrived during culture of the cells T, in Step S12, the controller 20 of the culture apparatus 10 causes the pump 100 to operate. Thus, the culture solution Sc in the culture vessel 12 starts to flow through the pipe 24 for measurement. Along therewith, the controller 20 instructs the cell density measurement device 18 to measure the cell density.

In Step S14, the light source 80 operates to emit the light L0 toward the culture solution Sc at the measurement point Pm in the pipe 24 for measurement. Next, in Step S 16, the first optical sensor 82 measures the intensity of the scattered light L1 scattered from the cells T suspended in the culture solution Sc present at the measurement point Pm in the pipe 24 for measurement.

FIG. 11A is a view for illustrating the culture solution in a state in which the cells are dispersed and when the first optical sensor measures the intensity of the scattered light.

As illustrated in FIG. 11A, when the first optical sensor 82 measures the intensity of the scattered light L1, the culture solution Sc is flowing through the pipe 24 for measurement at a predetermined certain flow velocity Vm due to the operation of the pump 100. Thus, the culture solution Sc in the pipe 24 for measurement is in a state in which the cells T are suspended. Accordingly, the light source 80 emits the light L0 to the culture solution Sc in the state in which the cells T are suspended. Further, the first optical sensor 82 measures the intensity of the scattered light L1 scattered from the cells T suspended in the culture solution Sc which the light L0 from the light source 80 has entered.

After the first optical sensor 82 ends the measurement of the intensity of the scattered light L1, in Step S18, the pump 100 is stopped. Thus, the flow of the culture solution Sc in the pipe 24 for measurement is stopped. With this stop of the pump 100, the cells T in the culture solution Sc precipitate at a position of the pipe 24 for measurement below the measurement point Pm. The pump 100 is a roller pump, and hence the culture solution Sc in the pipe 24 for measurement stays inside of the pipe 24 for measurement without returning to the culture vessel 12.

In Step S20, it is determined whether or not a predetermined precipitation time period of the cells T has elapsed from when the pump 100 has stopped. The predetermined precipitation time period is a time period required for the cells T to precipitate below the measurement point Pm of the pipe 24 for measurement, that is, a time period required until the cells T disappear from the culture solution Sc at the measurement point Pm. The predetermined precipitation time period can be experimentally obtained in advance. After the predetermined precipitation time period has elapsed, the process proceeds to next Step S22.

There is a case in which 100% of the cells do not precipitate depending on the type of the cells. In this case, a time period required until the movement of the cells in the pipe 24 for measurement substantially stops from the stop of the pump 100 is regarded as the precipitation time period.

In Step S22, the second optical sensor 84 measures the intensity of the transmitted light transmitted through the culture solution Sc present at the measurement point Pm in the pipe 24 for measurement.

FIG. 11B is a view for illustrating the culture solution in a state in which the cells are precipitated and when the second optical sensor measures the intensity of the transmitted light.

As illustrated in FIG. 11B, when the second optical sensor 84 measures the intensity of the transmitted light L2, the cells T precipitate below the measurement point Pm due to the stop of the pump 100. As a result, a culture solution Sc in which the cells T are not suspended (that is, the cells T do not substantially exist) is present at the measurement point Pm. Thus, the light source 80 emits the light L0 to the culture solution Sc in a state in which the cells T are not suspended. Further, the second optical sensor 84 measures the intensity of the transmitted light L2 transmitted through the culture solution Sc in the state in which the cells T are not suspended.

The reason why such precipitation of the cells T occurs is because a part of the pipe 24 for measurement in which the light source 80, the first optical sensor 82, and the second optical sensor 84 are provided extends at least in the vertical direction (Z axis direction).

After the second optical sensor 84 ends the measurement of the intensity of the transmitted light L2, in Step S24, the light source 80 stops emitting the light L0. Then, the measurement of the intensity of each of the scattered light L1 and the transmitted light L2 is completed.

Strictly speaking, the measurement timing of the intensity of the scattered light L1 and the measurement timing of the intensity of the transmitted light L2 are different from each other because the precipitation time period of the cells T is present therebetween. However, the precipitation time period of the cells T is several minutes and is a very short time period as compared to the total culture time period of the cells T. Accordingly, the measurement timing of the intensity of the scattered light L1 and the measurement timing of the intensity of the transmitted light L2 are substantially the same timing.

The transmitted light intensity ratio calculation unit 102 of the controller 86 of the cell density measurement device 18 calculates, based on the intensity of the transmitted light L2 measured by the second optical sensor 84 at a certain cell density measurement timing, a transmitted light intensity ratio of the culture solution Sc at this timing. That is, the second optical sensor 84 and the transmitted light intensity ratio calculation unit 102 form a transmitted light intensity ratio measurement unit of the culture apparatus 10 for measuring the transmitted light intensity ratio of the culture solution Sc. The transmitted light intensity ratio of the culture solution is a value representing, in percentage, a value obtained by dividing the intensity of the transmitted light exiting from the culture solution in which the cells are not suspended (in a state in which the cells are settled or a state in which a time period considered to be required to achieve settling has elapsed) by the intensity of the transmitted light of the unused culture solution in which the cells are not suspended.

Specifically, as described above and as illustrated in FIG. 11B, the second optical sensor 84 measures the intensity of the transmitted light L2 transmitted through the culture solution Sc in the state in which the cells T are not suspended (that is, part of the culture solution Sc in which the cells T are absent). Thus, the transmitted light intensity ratio calculation unit 102 calculates the transmitted light intensity ratio of the culture solution Sc in the state in which the cells T are not suspended (that is, the culture solution Sc alone).

The reason why the transmitted light intensity ratio of the culture solution Sc in the state in which the cells T are not suspended is measured (calculated) as described above is because the transmittance of the culture solution Sc is reduced as the culture progresses.

Specifically, the transmittance of the culture solution is reduced from the unused state (state before the culture start) depending on the type of the culture solution and the type of the cultured cells. For example, the transmittance of the culture solution is reduced due to, for example, a change over time of the culture solution itself, deposition of a component forming the culture solution, or a metabolite from the cell because of, for example, substances that do not contribute to scattering of light.

When such a change over time of the transmittance of the culture solution occurs, the measurement conditions at the time of optically measuring the cell density of this culture solution also change. For example, the measurement conditions of the cell density immediately after the culture start and the measurement conditions of the cell density immediately before the culture end may be greatly different from each other. As a result, during the cell culture, there is a possibility that the measurement accuracy of the cell density becomes unstable and the culture cannot be appropriately executed based on the cell density.

Accordingly, every time the cell measurement timing arrives, the transmitted light intensity ratio of the culture solution Sc at this timing is measured (calculated). Further, under a state in which the cells T are not suspended, the transmitted light intensity ratio of the culture solution Sc is measured (calculated).

When the transmittance of the culture solution Sc at an n-th (integer) cell density measurement timing is represented by $\tau(n)$, the intensity of the transmitted light measured by the second sensor 84 at this timing is represented by $It(n)$, and the intensity of the light of the light source 80 is represented by Ic, a relationship expressed by Equation 1 is obtained.

[Math. 1]

$$It(n) = \tau(n) \times Ic \quad \cdot \cdot \cdot \quad (\text{Equation 1})$$

Further, when transmittance of a culture solution Sc in a state in which the cells T are not suspended and immediately before the culture start is represented by $\tau(0)$ and an intensity of light transmitted through this culture solution Sc is represented by $It(0)$, a relationship expressed by Equation 2 is obtained. The "culture solution Sc immediately before the culture start" as referred to here refers to a culture solution in a substantially unused state and, for example, before the agitation of the agitation device 14 is started.

[Math. 2]

$$It(0) = \tau(0) \times Ic \quad \cdot \cdot \cdot \quad (\text{Equation 2})$$

The light source 80 outputs light of a certain intensity Ic, and hence Equation 3 below can be obtained from Equation 1 and Equation 2.

[Math. 3]

$$\frac{\tau(n)}{\tau(0)} = \frac{It(n)}{It(0)} = R(n) \quad \cdot \cdot \cdot \quad (\text{Equation 3})$$

$It(n)/It(0)$ represents the transmitted light intensity ratio which is a ratio of the intensity of the transmitted light of the culture solution during cell culture and in a state in which the cells are not suspended to the intensity of the transmitted light of the unused culture solution. The transmitted light intensity ratio is hereinafter represented by $R(n)$. When the transmittance $\tau(0)$ at the time when the culture solution Sc is in the unused state (that is, the transmittance of the culture solution Sf) is represented by averaged transmittance $\tau(0)'$ among a plurality of culture solutions used when a calibration curve is created, Equation 3 is replaced by Equation 4. Details of the calibration curve referred to here are described later.

[Math. 4]

$$\frac{\tau(n)}{\tau(0)'} = \frac{It(n)}{It(0)'} = R(n) \quad \cdot \cdot \cdot \quad (\text{Equation 4})$$

In the case of the first embodiment, the transmitted light intensity ratio calculation unit 102 calculates the transmitted light intensity ratio at the cell density measurement timing through use of Equation 4.

That is, the transmitted light intensity ratio calculation unit 102 calculates the transmitted light intensity ratio $R(n)$ of the culture solution Sc at the n-th (integer) cell density measurement timing from the intensity $It(n)$ of the transmitted light L2 transmitted through the culture solution Sc in the state in which the cells T are not suspended, which has been measured by the second optical sensor 84 at this timing, and the averaged intensity $It(0)'$ of the transmitted light L2 measured by the second optical sensor 84 after being transmitted through a plurality of culture solutions Sc used when the calibration curve of the cells T is created.

When expansion culture is performed, the amount of the culture solution Sc immediately before the culture start is very small, and, in some cases, the inside of the pipe 24 for measurement cannot be filled with the culture solution Sc. In those cases, the transmitted light intensity ratio of the culture solution Sc may be calculated through use of the intensity of the transmitted light of the culture solution Sf before being supplied into the culture vessel 12.

FIG. 12 is view for illustrating a modification mode of the culture apparatus according to the first embodiment.

As illustrated in FIG. 12, when the expansion culture is performed, a second light source 108 and a third optical sensor 110 are provided to the pipe 22 for supply for supplying the unused culture solution Sf into the culture vessel 12. The second light source 108 emits light to the culture solution Sf in the pipe 22 for supply, that is, the culture solution Sf in which the cells T are absent. The third optical sensor 110 is opposed to the second light source 108 across the culture solution Sf in the pipe 22 for supply, and measures an intensity of transmitted light transmitted through the culture solution Sf. The intensity of the transmitted light measured by this third optical sensor 110 is used as It(0) in Equation 1. In this case, it is preferred that the second light source 108 be the same as the first light source 80, and it is also preferred that the third optical sensor 110 be the same as the second optical sensor 84.

The cell density calculation unit 104 of the controller 86 of the cell density measurement device 18 further calculates, based on the transmitted light intensity ratio calculated by the transmitted light intensity ratio calculation unit 102 at a certain cell density measurement timing and the intensity of the scattered light measured by the first optical sensor 82 at this timing, the cell density of the culture solution Sc at this timing.

Specifically, the cell density calculation unit 104 uses an equation of the calibration curve indicating the relationship between the cell density of the culture solution and the intensity of the scattered light exiting from this culture solution.

FIG. 13 is a graph for showing an example of the calibration curve indicating the relationship between the cell density of the culture solution and the intensity of the scattered light exiting from this culture solution.

The calibration curve indicated by the broken line of FIG. 13 is created in advance as many as the number of combinations, such as the types of the cells and the components of the culture solution. For example, the calibration curve is created in advance by measuring the intensity of the transmitted light exiting from each of a plurality of different culture solutions having the same component and known cell densities. The culture solutions used when the calibration curve is created are substantially in an unused state, and the transmittances thereof are substantially constant. For example, different numbers of cells are suspended in the respective unused culture solutions. Thus, a plurality of unused culture solutions having different cell densities can be created. The intensities of the scattered light beams exiting from such culture solutions are measured by substantially the same measurement conditions as the measurement conditions of the cell density measurement device 18. For example, the light source 80, the first optical sensor 82, and the second optical sensor 84 are used in the same layout. Thus, the calibration curve to be used by the cell density calculation unit 104 can be obtained. That is, a calibration curve in a case in which the transmittance of the culture solution does not change over time from the initial state can be obtained.

The cell density calculation unit 104 calculates the cell density through use of the equation expressing the calibration curve. As shown in FIG. 13, the equation expressing the calibration curve is calculated from a plurality of combinations (measurement points m1 to m5) of the cell density and the intensity of the scattered light by using the least squares method. Further, the equation expressing the calibration curve is stored in the storage device of the controller 86. The equation expressing the calibration curve may be expressed as Equation 5 below.
[Math. 5]

$$\mathbf{Is} = \mathbf{a} \times \mathbf{C} + \mathbf{b} \quad \cdots \quad (\text{Equation } 5)$$

In Equation 5, a variable Is represents the intensity of the scattered light L1 measured by the first optical sensor 82. Further, a variable C represents the cell density of the culture solution Sc. Further, each of "a" and "b" represents a constant coefficient.

Thus, an equation for obtaining the cell density C by the intensity Is of the scattered light L1 can be expressed as Equation 6.
[Math. 6]

$$\mathbf{C} = \frac{1}{\mathbf{a}}(\mathbf{Is} - \mathbf{b}) \cdots \quad (\text{Equation } 6)$$

The equation expressed in Equation 6, that is, the coefficients "a" and "b" are calculated under a condition in which the transmittance of the culture solution does not change over time from the initial state. Accordingly, the intensity of the scattered light L1 measured by the first optical sensor 82 cannot be directly substituted into the variable Is.

Specifically, the intensity of the scattered light L1 measured by the first optical sensor 82 during culture is affected by the change over time of the transmittance of the culture solution Sc. That is, light before reaching a cell and light scattered from the cell are transmitted through the culture solution Sc, and hence the intensity of the scattered light L1 is affected by the transmittance of the culture solution Sc. As a result, the intensity of the scattered light L1 at the early stage of the culture and the intensity of the scattered light L1 at the later stage of the culture are affected by different transmittances of the culture solution Sc.

In view of the above, the cell density calculation unit 104 corrects the intensity of the scattered light L1 measured by the first optical sensor 82 at a certain cell density measurement timing through use of the transmitted light intensity ratio of the culture solution Sc measured (calculated) at this timing. Thus, the influence of the change over time of the transmittance of the culture solution Sc included in the intensity of the scattered light L1 measured at this timing is canceled.

Specifically, as expressed in Equation 7, the cell density calculation unit 104 divides the intensity of the scattered light L1 measured by the first optical sensor 82 at a certain cell density measurement timing by the transmitted light intensity ratio of the culture solution Sc measured (calculated) at this timing.

[Math. 7]

$$Is' = \frac{Is(n)}{R(n)} \quad \cdots \quad (Equation\ 7)$$

In Equation 7, Is' represents the corrected intensity of the scattered light L1. When Equation 7 is substituted into Equation 6, Equation 8 below can be obtained.
[Math. 8]

$$C(n) = \frac{1}{a}\left(\frac{Is(n)}{R(n)} - b\right) \quad \cdots \quad (Equation\ 8)$$

The cell density calculation unit 104 calculates, through use of the relationship equation expressed in Equation 8, from the intensity Is(n) of the scattered light L1 measured by the first optical sensor 82 at the n-th cell density measurement timing and the transmitted light intensity ratio R(n) of the culture solution Sc measured (calculated) at this timing, the cell density C(n) at this timing. Thus, the cell density calculation unit 104 can calculate, during the cell culture, the cell density whose influence of the change over time of the transmittance of the culture solution Sc is canceled. As a result, the cell density measurement device 18 can measure the cell density with a stable accuracy during cell culture.

When an air bubble is present in the pipe 24 for measurement, the air bubble detection unit 106 of the controller 86 of the cell density measurement device 18 detects this air bubble. The "air bubble" as used herein refers to an air bubble that adheres to an inner peripheral surface of the pipe 24 for measurement and substantially does not move.

When an air bubble is present in the pipe 24 for measurement, light scattering occurs at this air bubble. As a result, scattered light from the cell and scattered light from the air bubble enter the first optical sensor 82. As a result, the calculation accuracy of the cell density calculated by the cell density calculation unit 104 is affected.

The air bubble detection unit 106 detects the presence of the air bubble based on the intensity of the scattered light L1 measured by the first optical sensor 82. Specifically, when the intensity of the scattered light L1 measured by the first optical sensor 82 is larger than the intensity of the scattered light L1 measured at a timing immediately before to exceed a predetermined threshold value, the air bubble detection unit 106 determines that an air bubble is present. It is preferred that the measurement timing of the first optical sensor 82 for determining the presence of the air bubble be a timing immediately before the cell density measurement timing.

When the air bubble detection unit 106 has detected an air bubble, an operation of removing the air bubble from the inside of the pipe 24 for measurement is executed. Specifically, an air bubble detection signal is transmitted from the controller 86 of the cell density measurement device 18 to the controller 20 of the culture apparatus 10. When this air bubble detection signal is received, the controller 20 controls the pump 100 to discharge the air bubble in the pipe 24 for measurement to the outside.

In the case of the first embodiment, the pipe 24 for measurement is connected to the culture vessel 12 so that its inlet end 24c enters the culture solution Sc in the culture vessel 12 and its outlet end 24d is separated away from a liquid surface of the culture solution Sc. Further, in the case of the first embodiment, the pump 100 is a roller pump capable of performing a forward rotation operation and a reverse rotation operation. When the pump 100 performs the forward rotation operation, a flow of the culture solution Sc from the inlet end 24c to the outlet end 24d is caused in the pipe 24 for measurement. When the pump 100 performs the reverse rotation operation, a flow of the culture solution Sc from the outlet end 24d to the inlet end 24c is caused.

When an air bubble is present in the pipe 24 for measurement, the pump 100 executes the reverse rotation operation to introduce air into the pipe 24 for measurement via the outlet end 24d. Thus, the culture solution Sc including the air bubble in the pipe 24 for measurement is replaced by air. After this replacement is completed, the pump 100 executes the forward rotation operation to supply the culture solution Sc in the culture vessel 12 into the pipe 24 for measurement. Thus, the air bubble in the pipe 24 for measurement is discharged to the outside.

The air bubble in the pipe 24 for measurement can be discharged to the outside by other methods.

For example, when the air bubble detection unit 106 detects an air bubble, the pump 100 may alternately repeat the forward rotation operation and the reverse rotation operation. Thus, a pulsating flow of the culture solution Sc is caused in the pipe 24 for measurement, and hence the air bubble adhering to the inner peripheral surface of the pipe 24 for measurement is separated from this inner peripheral surface. As a result, the air bubble in the pipe 24 for measurement is discharged to the outside.

Further, for example, when the air bubble detection unit 106 detects an air bubble, the pump 100 may supply the culture solution Sc into the pipe 24 for measurement at a flow velocity slower than the flow velocity Vm used when the first optical sensor 82 measures the intensity of the scattered light L1 at the cell density measurement timing. When the culture solution Sc is supplied at a flow velocity slower than the flow velocity at which the air bubble has been generated, while generation of a new air bubble is suppressed, the air bubble present in the pipe 24 for measurement can be pushed out to the outside.

According to the first embodiment as described above, during the cell culture, the measurement of the cell density can be frequently executed with a stable accuracy. That is, the cell density can be measured without requiring human power and in consideration of the change over time of the transmittance of the culture solution. Thus, the execution frequency of the cell density measurement can be increased and the stability of the measurement accuracy is improved.

(Second Embodiment)

A second embodiment is different from the above-mentioned first embodiment in the cell density measurement device. Thus, the second embodiment is described mainly on the differences. Components of the second embodiment substantially the same as those of the above-mentioned first embodiment are denoted by the same reference symbols.

FIG. 14A is a view for illustrating a culture solution in a state in which cells are dispersed and when an optical sensor measures an intensity of transmitted light in a culture apparatus according to the second embodiment. Further, FIG. 14B is a view for illustrating the culture solution in a state in which the cells are precipitated and when the optical sensor measures an intensity of transmitted light in the culture apparatus according to the second embodiment.

As illustrated in FIG. 14A and FIG. 14B, in the case of the second embodiment, a cell density measurement device 218 includes one light source 80 and one optical sensor 282, unlike the cell density measurement device 18 of the above-mentioned first embodiment. The light source 80 and the optical sensor 282 are opposed to each other across the culture solution Sc in the pipe 24 for measurement.

In the case of the second embodiment, the cell density measurement device 218 includes no optical sensor for measuring an intensity of scattered light. Thus, the cell density is measured (calculated) based on transmitted light measured by the optical sensor 282.

First, as illustrated in FIG. 14A, the optical sensor 282 measures an intensity of transmitted light L3 transmitted through the culture solution Sc in a state in which the cells T are suspended. Further, as illustrated in FIG. 14B, the optical sensor 282 measures an intensity of transmitted light L4 transmitted through the culture solution Sc in a state in which the cells are not suspended.

A transmitted light intensity ratio calculation unit of a controller of the cell density measurement device 218 calculates, similarly to the transmitted light intensity ratio calculation unit 102 of the above-mentioned first embodiment, based on the transmitted light L4 measured by the optical sensor 282 at a cell density measurement timing, that is, the intensity of the transmitted light L4 transmitted through the culture solution Sc in the state in which the cells are not suspended, a transmitted light intensity ratio of the culture solution Sc at this timing.

Further, in the case of the second embodiment, a calibration curve indicating a relationship between a cell density of a culture solution and an intensity of transmitted light exiting from this culture solution is created in advance. The calibration curve of the second embodiment is also created in advance under a condition in which the culture solution is in an unused state, similarly to the calibration curve of the above-mentioned first embodiment. An equation expressing the calibration curve can be expressed as Equation 9 below.
[Math. 9]

$$It1 = d \times C + e \quad \cdots \quad (Equation\ 9)$$

A variable It1 represents the intensity of the transmitted light L3 measured by the first optical sensor 82. Further, a variable C represents the cell density of the culture solution Sc. Further, each of "d" and "e" represents a constant coefficient.

The transmitted light intensity ratio of the culture solution Sc at the cell density timing is also calculated similarly to the above-mentioned first embodiment. The transmitted light intensity ratio R(n) of the culture solution Sc at the n-th cell density measurement timing is calculated by an equation expressed by Equation 10.
[Math. 10]

$$R(n) = \frac{It2(n)}{It2(0)} \quad \cdots \quad (Equation\ 10)$$

It2(n) in Equation 10 represents the intensity of the transmitted light L4 measured at the n-th cell density measurement timing. Further, It2(0) represents an intensity of light transmitted through the culture solution Sc in a state in which the cells T are not suspended and immediately before the culture start.

Further, similarly to the intensity of the scattered light L1 in the above-mentioned first embodiment, the intensity of the transmitted light L3 measured by the optical sensor 282 at the cell density measurement timing is also corrected based on the transmitted light intensity ratio of the culture solution Sc at this timing.

Specifically, as expressed in Equation 11, the intensity of the transmitted light L3 measured by the optical sensor 282 at a certain cell density measurement timing is divided by the transmitted light intensity ratio of the culture solution Sc measured (calculated) at this timing.
[Math. 11]

$$It' = \frac{It1(n)}{R(n)} \quad \cdots \quad (Equation\ 11)$$

In Equation 11, It' represents the corrected intensity of the transmitted light L3. When Equation 11 is substituted into Equation 9, Equation 12 below can be obtained.
[Math. 12]

$$C(n) = \frac{1}{d}\left(\frac{It1(n)}{R(n)} - e\right) \cdots \quad (\text{Equation 12})$$

A cell density calculation unit of the cell density measurement device 218 calculates, through use of the relationship equation expressed in Equation 12, from the intensity $It1(n)$ of the transmitted light L3 measured by the optical sensor 282 at the n-th cell density measurement timing and the transmitted light intensity ratio $R(n)$ of the culture solution Sc measured (calculated) at this timing, the cell density $C(n)$ at this timing. Thus, during the cell culture, the cell density whose influence of the change over time of the transmittance of the culture solution Sc is canceled can be calculated. As a result, the cell density measurement device 218 can measure the cell density with a stable accuracy during cell culture.

Similarly to the above-mentioned first embodiment, also in the second embodiment as described above, during the cell culture, the measurement of the cell density can be frequently executed with a stable accuracy.

The present disclosure has been described above by means of the above-mentioned embodiments, but the embodiments of the present disclosure are not limited thereto.

For example, in the case of the above-mentioned first embodiment, the cell density measurement device 18 measures the cell density of the culture solution Sc in the pipe 24 for measurement. However, the embodiment of the present disclosure is not limited thereto. The cell density measurement device can also measure the cell density of the culture solution in the culture vessel. In this case, in order to measure the intensity of light exiting from the culture solution in which the cells are not dispersed, the agitation of the culture solution is temporarily stopped.

Further, in the case of the above-mentioned first embodiment, the culture apparatus 10 executes so-called expansion culture in which the amount of the culture solution is increased as the culture progresses. However, the embodiment of the present disclosure is not limited thereto. The culture apparatus according to an embodiment of the present disclosure may be a culture apparatus for performing culture through use of a constant amount of culture solution.

Moreover, in the case of the above-mentioned first embodiment, as illustrated in FIG. 8, the first optical sensor 82 and the second optical sensor 84 are used to measure the cell density. Those first optical sensor 82 and second optical sensor 84 may be the same optical sensor, or may be different optical sensors. Further, when there is a large intensity difference (for example, an intensity difference causing a difference in range) between the intensity of the scattered light L1 measured by the first optical sensor 82 and the intensity of the transmitted light L2 measured by the second optical sensor 84, an optical element such as a filter may be used. Even when the respective measurement errors of the first optical sensor 82 and the second optical sensor 84 are substantially the same, when the range of the intensity of light entering each optical sensor is different, this error may greatly affect the calculation accuracy of the cell density. As a countermeasure thereagainst, for example, an optical element such as a filter for reducing the intensity, which changes the intensity of the transmitted light L2 entering the second optical sensor 84, may be arranged between the second optical sensor 84 which the transmitted light L2 having a relatively high intensity enters and the culture solution Sc.

That is, in a broad sense, a culture apparatus according to an embodiment of the present disclosure includes: a culture vessel configured to accommodate a culture solution for culturing cells; a light source configured to emit light toward the culture solution; a first optical sensor configured to measure an intensity of light exiting from the culture solution; a transmitted light intensity ratio measurement unit configured to measure a transmitted light intensity ratio which is a ratio of an intensity of transmitted light of the culture solution during cell culture and in a state in which the cells are not suspended to an intensity of transmitted light of an unused culture solution; and a cell density calculation unit configured to calculate a cell density of the culture solution. The cell density calculation unit is configured to, when the cell density calculation unit calculates the cell density at a certain timing during the cell culture: correct the intensity of the light exiting from the culture solution in a state in which the cells are suspended and being measured by the first optical sensor at the certain timing through use of the transmitted light intensity ratio measured by the transmitted light intensity ratio measurement unit at the certain timing; and calculate the cell density at the certain timing based on the corrected intensity of the light and an equation of a calibration curve indicating a relationship between a cell density of a culture solution and an intensity of light exiting from the culture solution, the calibration curve being created in advance under a condition in which the culture solution is in an unused state.

Further, in a broad sense, a cell density measurement device according to an embodiment of the present disclosure includes: a light source configured to emit light toward the culture solution; an optical sensor configured to measure an intensity of light exiting from the culture solution; a transmitted light intensity ratio measurement unit configured to measure a transmitted light intensity ratio which is a ratio of an intensity of transmitted light of the culture solution during cell culture and in a state in which the cells are not suspended to an intensity of transmitted light of an unused culture solution; and a cell density calculation unit configured to calculate a cell density of the culture solution. The cell density calculation unit is configured to, when the cell density calculation unit calculates the cell density at a certain timing during the cell culture: correct the intensity of the light exiting from the culture solution in a state in which the cells are suspended and being measured by the optical sensor at the certain timing through use of the transmitted light intensity ratio measured by the transmitted light intensity ratio measurement unit at the certain timing; and calculate the cell density at the certain timing based on the corrected intensity of the light and an equation of a calibration curve indicating a relationship between a cell density of a culture solution and an intensity of light exiting from the culture solution, the calibration curve being created in advance under a condition in which the culture solution is in an unused state.

In addition, in a broad sense, a cell density measurement method according to an embodiment of the present disclosure is a cell density measurement method for measuring a cell density of a culture solution during cell culture, the cell density measurement method including: emitting, by a light source, light toward the culture solution; measuring, by an optical sensor, an intensity of light exiting from the culture solution; measuring a transmitted light intensity ratio which is a ratio of an intensity of transmitted light of the culture solution during the cell culture and in a state in which cells are not suspended to an intensity of transmitted light of an unused culture solution; and when calculating the cell density at a certain timing during the cell culture, correcting the intensity of the light exiting from the culture solution in a state in which the cells are suspended and being measured by the optical sensor at the certain timing through use of the transmitted light intensity ratio measured at the certain timing; and calculating the cell density at the certain timing based on the corrected intensity of the light and an equation of a calibration curve indicating a relationship between a cell density of a culture solution and an intensity of light exiting from the culture solution, the calibration curve being created in advance under a condition in which the culture solution is in an unused state.

[Industrial Applicability]

The present disclosure is applicable to cell culture performed while the cell density is monitored.

**Claims**

1. A culture apparatus comprising:

a culture vessel configured to accommodate a culture solution for culturing cells;
a light source configured to emit light toward the culture solution;
a first optical sensor configured to measure an intensity of light exiting from the culture solution;
a transmitted light intensity ratio measurement unit configured to measure a transmitted light intensity ratio which is a ratio of an intensity of transmitted light of the culture solution during cell culture and in a state in which the cells are not suspended to an intensity of transmitted light of an unused culture solution; and
a cell density calculation unit configured to calculate a cell density of the culture solution,
wherein the cell density calculation unit is configured to, when the cell density calculation unit calculates the cell density at a certain timing during the cell culture:

correct the intensity of the light exiting from the culture solution in a state in which the cells are suspended and being measured by the first optical sensor at the certain timing through use of the transmitted light intensity ratio measured by the transmitted light intensity ratio measurement unit at the certain timing; and
calculate the cell density at the certain timing based on the corrected intensity of the light and an equation of a calibration curve indicating a relationship between a cell density of a culture solution and an intensity of light exiting from the culture solution, the calibration curve being created in advance under a condition in which the culture solution is in an unused state.

2. The culture apparatus according to claim 1,

wherein the first optical sensor is configured to measure an intensity of scattered light scattered from the cells suspended in the culture solution which the light from the light source has entered,
wherein the transmitted light intensity ratio measurement unit includes:

a second optical sensor opposed to the light source across the culture solution, the second optical sensor being configured to measure an intensity of transmitted light transmitted through the culture solution in the state in which the cells are not suspended; and
a transmitted light intensity ratio calculation unit configured to calculate the transmitted light intensity ratio of the culture solution based on the intensity of the transmitted light measured by the second optical sensor,

wherein the equation of the calibration curve is an equation indicating a relationship between a cell density of a culture solution and an intensity of scattered light exiting from the culture solution, the equation being created in advance under a condition in which the culture solution is in an unused state, and

wherein the cell density calculation unit is configured to correct the intensity of the scattered light by dividing the intensity of the scattered light by the transmitted light intensity ratio calculated by the transmitted light intensity ratio calculation unit.

3. The culture apparatus according to claim 2, wherein the transmitted light intensity ratio calculation unit is configured to calculate the transmitted light intensity ratio at the certain timing by dividing the intensity of the transmitted light measured by the second optical sensor at the certain timing by the intensity of the transmitted light measured by the second optical sensor after being transmitted through a culture solution in a state in which the cells are not suspended and immediately before culture start.

4. The culture apparatus according to claim 2, further comprising:

a pipe for supply through which the unused culture solution is supplied to the culture vessel;
a second light source configured to emit light to the culture solution in the pipe for supply; and
a third optical sensor opposed to the second light source across the culture solution in the pipe for supply, the third optical sensor being configured to measure an intensity of transmitted light transmitted through the culture solution in the pipe for supply,
wherein the transmitted light intensity ratio calculation unit is configured to calculate the transmitted light intensity ratio at the certain timing by dividing the intensity of the transmitted light measured by the second optical sensor at the certain timing by the intensity of the transmitted light measured by the third optical sensor.

5. The culture apparatus according to any one of claims 2 to 4, further comprising:

a pipe for measurement connected to the culture vessel, the pipe for measurement including a part extending in a direction including at least a vertical direction component, the part passing between the light source and the first optical sensor and between the light source and the second optical sensor; and
a pump configured to supply the culture solution in the culture vessel into the pipe for measurement,
wherein the light source is configured to emit light toward a measurement point in the pipe for measurement,
wherein the second optical sensor is configured to measure the intensity of the transmitted light transmitted through the culture solution present at the measurement point, under a state in which the cells precipitate at a position in the pipe for measurement below the measurement point due to stop of the pump, and
wherein the first optical sensor is configured to measure the intensity of the scattered light scattered from the cells suspended in the culture solution present at the measurement point, under a state in which the culture solution flows through the pipe for measurement due to an operation of the pump.

6. The culture apparatus according to claim 5,

wherein the pipe for measurement is a tube that is transparent and has flexibility,
wherein the culture apparatus further comprises a clamp mechanism configured to sandwich the tube to form an incident surface and an exit surface in the tube, the incident surface and the exit surface being formed in parallel to each other and each having a planar shape,
wherein the light source is configured to emit light toward the incident surface of the tube, and
wherein the first optical sensor and the second optical sensor are configured to receive scattered light and transmitted light, respectively, exiting from the exit surface of the tube.

7. The culture apparatus according to claim 5 or 6, further comprising an air bubble detection unit configured to determine that an air bubble is present inside of the pipe for measurement when the intensity of the scattered light measured by the first optical sensor is larger than the intensity of the scattered light measured at a timing immediately before to exceed a predetermined threshold value.

8. The culture apparatus according to claim 7,

wherein the pump is configured to perform a forward rotation operation and a reverse rotation operation,
wherein the pipe for measurement is connected to the culture vessel so that an inlet end thereof enters the culture solution and an outlet end thereof is separated away from a liquid surface of the culture solution, and
wherein the pump is configured to execute, when the air bubble detection unit determines that the air bubble is present in the pipe for measurement, the reverse rotation operation to introduce air into the pipe for measurement via the outlet end so that the culture solution in the pipe for measurement is replaced by the air, and execute the forward rotation operation after the replacement is completed to supply the culture solution into the pipe for measurement.

**9.** The culture apparatus according to claim 7,

wherein the pump is configured to perform a forward rotation operation and a reverse rotation operation, and
wherein the pump is configured to alternately repeat, when the air bubble detection unit determines that the air bubble is present in the pipe for measurement, the forward rotation operation and the reverse rotation operation so that a pulsating flow of the culture solution is caused in the pipe for measurement.

**10.** The culture apparatus according to claim 7, wherein the pump is configured to supply, when the air bubble detection unit determines that the air bubble is present in the pipe for measurement, the culture solution into the pipe for measurement at a flow velocity slower than a flow velocity used when the first optical sensor measures the intensity of the scattered light.

**11.** The culture apparatus according to any one of claims 5 to 10,

wherein an optical axis of the light source and an optical axis of the second optical sensor match each other,
wherein an optical axis of the first optical sensor intersects with the optical axis of the light source, and
wherein a distance from an intersection between the optical axis of the light source and the optical axis of the first optical sensor to a light receiving surface of the second optical sensor is a distance of ±20% or less with respect to a distance from the intersection to a light receiving surface of the first optical sensor.

**12.** The culture apparatus according to claim 11, wherein an angle formed between the optical axis of the light source and the optical axis of the first optical sensor is an angle falling within a range of from 0 degrees to 90 degrees.

**13.** The culture apparatus according to any one of claims 2 to 12, further comprising an optical element arranged between the second optical sensor and the culture solution, the optical element being configured to change the intensity of the transmitted light entering the second optical sensor.

**14.** The culture apparatus according to claim 1,

wherein the first optical sensor is opposed to the light source across the culture solution, and is configured to measure an intensity of transmitted light transmitted through the culture solution in the state in which the cells are suspended,

wherein the transmitted light intensity ratio measurement unit includes a transmitted light intensity ratio calculation unit configured to calculate a transmitted light intensity ratio of the culture solution based on the intensity of the transmitted light transmitted through the culture solution in the state in which the cells are not suspended and measured by the first optical sensor,
wherein the equation of the calibration curve is an equation indicating a relationship between a cell density of a culture solution and an intensity of transmitted light exiting from the culture solution, the equation being created in advance under a condition in which the culture solution is in an unused state, and
wherein the cell density calculation unit is configured to correct the intensity of the transmitted light by dividing the intensity of the transmitted light by the transmitted light intensity ratio calculated by the transmitted light intensity ratio calculation unit.

**15.** The culture apparatus according to any one of claims 1 to 14, further comprising a culture solution supply control unit configured to control a supply rate of the unused culture solution to be supplied to the culture vessel,

wherein the culture solution supply control unit is configured to control the supply rate of the culture solution based on the cell density calculated by the cell density calculation unit.

**16.** The culture apparatus according to claim 15, further comprising a cell growth rate calculation unit configured to calculate a cell growth rate based on the cell density calculated by the cell density calculation unit at each of two successive timings,

wherein the culture solution supply control unit is configured to control the supply rate of the culture solution based on the cell growth rate calculated by the cell growth rate calculation unit.

**17.** The culture apparatus according to claim 15 or 16, further comprising:

a culture solution supply schedule creation unit configured to create a culture solution supply schedule based on a doubling time of the cells, a target cell number, and a cell density profile indicating a suitable change over time of the cell density; and
a culture solution supply schedule correction unit configured to correct the culture solution supply schedule based on the cell density calculated by the cell density calculation unit,
wherein the culture solution supply control unit is configured to control the supply rate of the culture solution in accordance with the culture solution supply schedule.

**18.** A cell density measurement device comprising:

a light source configured to emit light toward the culture solution;
an optical sensor configured to measure an intensity of light exiting from the culture solution;
a transmitted light intensity ratio measurement unit configured to measure a transmitted light intensity ratio which is a ratio of an intensity of transmitted light of the culture solution during cell culture and in a state in which the cells are not suspended to an intensity of transmitted light of an unused culture solution; and
a cell density calculation unit configured to calculate a cell density of the culture solution,
wherein the cell density calculation unit is configured to, when the cell density calculation unit calculates the cell density at a certain timing during the cell culture:

correct the intensity of the light exiting from the culture solution in a state in which the cells are suspended and being measured by the optical sensor at the certain timing through use of the transmitted light intensity ratio measured by the transmitted light intensity ratio measurement unit at the certain timing; and
calculate the cell density at the certain timing based on the corrected intensity of the light and an equation of a calibration curve indicating a relationship between a cell density of a culture solution and an intensity of light exiting from the culture solution, the calibration curve being created in advance under a condition in which the culture solution is in an unused state.

**19.** A cell density measurement method for measuring a cell density of a culture solution during cell culture, the cell density measurement method comprising:

emitting, by a light source, light toward the culture solution;
measuring, by an optical sensor, an intensity of light exiting from the culture solution;
measuring a transmitted light intensity ratio which is a ratio of an intensity of transmitted light of the culture solution during the cell culture and in a state in which cells are not suspended to an intensity of transmitted light of an unused culture solution; and
when calculating the cell density at a certain timing during the cell culture,

correcting the intensity of the light exiting from the culture solution in a state in which the cells are suspended and being measured by the optical sensor at the certain timing through use of the transmitted light intensity ratio measured at the certain timing; and
calculating the cell density at the certain timing based on the corrected intensity of the light and an equation of a calibration curve indicating a relationship between a cell density of a culture solution and an intensity of light exiting from the culture solution, the calibration curve being created in advance under a condition in which the culture solution is in an unused state.

# FIG. 1

# FIG. 2

10

20 — CONTROLLER

CULTURE CONDITION
ACQUISITION UNIT — 64

CULTURE SOLUTION SUPPLY
SCHEDULE CREATION UNIT — 66

CULTURE SOLUTION SUPPLY
SCHEDULE CORRECTION UNIT — 68

CULTURE SOLUTION
SUPPLY CONTROL UNIT — 70

CELL GROWTH RATE
CALCULATION UNIT — 72

74 — INPUT DEVICE

AGITATION DEVICE — 14

CULTURE SOLUTION SUPPLY DEVICE — 16

OUTPUT DEVICE — 76

PUMP — 100

18
CELL DENSITY MEASUREMENT DEVICE

CONTROLLER — 86

TRANSMITTED LIGHT INTENSITY
RATIO CALCULATION UNIT — 102

CELL DENSITY
CALCULATION UNIT — 104

AIR BUBBLE
DETECTION UNIT — 106

82 — FIRST OPTICAL SENSOR

84 — SECOND OPTICAL SENSOR

LIGHT SOURCE — 80

# FIG. 3

# FIG. 4

# FIG. 5

# FIG. 6

CELL DENSITY

TIME

# FIG. 7

CELL DENSITY
(cells/ml)

CUMULATIVE
SUPPLY
AMOUNT
(ml)

TIME
(h)

CULTURE
END

# FIG. 8

# FIG. 9

# FIG. 10

START

S10 CELL MEASUREMENT TIMING HAS ARRIVED? — NO

YES

OPERATE PUMP ~S12

EMIT, BY LIGHT SOURCE, LIGHT TO CULTURE SOLUTION ~S14

MEASURE, BY FIRST OPTICAL SENSOR, INTENSITY OF SCATTERED LIGHT ~S16

STOP PUMP ~S18

S20 PREDETERMINED PRECIPITATION TIME PERIOD HAS ELAPSED? — NO

YES

MEASURE, BY SECOND OPTICAL SENSOR, INTENSITY OF TRANSMITTED LIGHT ~S22

STOP EMISSION OF LIGHT OF LIGHT SOURCE ~S24

END

# FIG. 11A

# FIG. 11B

# FIG. 12

# FIG. 13

# FIG. 14A

# FIG. 14B

## INTERNATIONAL SEARCH REPORT

| International application No. |
| --- |
| **PCT/JP2023/004809** |

### A. CLASSIFICATION OF SUBJECT MATTER

*C12Q 1/06*(2006.01)i; *C12M 1/00*(2006.01)i; *C12M 1/34*(2006.01)i
FI: C12M1/00 C; C12M1/34 A; C12Q1/06

According to International Patent Classification (IPC) or to both national classification and IPC

### B. FIELDS SEARCHED

Minimum documentation searched (classification system followed by classification symbols)

C12Q1/06; C12M1/00-3/10

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| X | WO 2016/063364 A1 (HITACHI HIGH-TECHNOLOGIES CORP.) 28 April 2016 (2016-04-28) claims, paragraphs [0015]-[0080], fig. 1-10 | 1-6, 11-19 |
| Y | claims, paragraphs [0015]-[0080], fig. 1-10 | 7, 10 |
| A | claims, paragraphs [0015]-[0080], fig. 1-10 | 8–9 |
| Y | JP 63-44150 A (KOKEN CO., LTD.) 25 February 1988 (1988-02-25) p. 2, upper left column, line 8 to lower left column, line 5 | 7, 10 |
| A | entire text | 1-6, 8-9, 11-19 |
| Y | JP 2021-101153 A (OHDAIRA LABORATORY CO.) 08 July 2021 (2021-07-08) paragraphs [0023]-[0025] | 7, 10 |
| A | entire text | 1-6, 8-9, 11-19 |
| Y | Microfilm of the specification and drawings annexed to the request of Japanese Utility Model Application No. 84435/1985 (Laid-open No. 808/1986) (KURIHARA KOGYO CO., LTD.) 07 January 1986 (1986-01-07), p. 3, lines 3, 4, p. 6, lines 13-19 | 7, 10 |
| A | p. 3, lines 3, 4, p. 6, lines 13-19 | 1-6, 8-9, 11-19 |

☑ Further documents are listed in the continuation of Box C.   ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **12 April 2023** | **25 April 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

# EP 4 491 738 A1

**INTERNATIONAL SEARCH REPORT**

International application No.

**PCT/JP2023/004809**

### C. DOCUMENTS CONSIDERED TO BE RELEVANT

| Category* | Citation of document, with indication, where appropriate, of the relevant passages | Relevant to claim No. |
|---|---|---|
| A | WO 2016/013395 A1 (HITACHI HIGH-TECHNOLOGIES CORP.) 28 January 2016 (2016-01-28)<br> entire text | 1-19 |
| A | JP 2012-217426 A (TAITEC CORP.) 12 November 2012 (2012-11-12)<br> entire text | 1-19 |
| A | JP 2004-101196 A (HAMAMATSU PHOTONICS K.K.) 02 April 2004 (2004-04-02)<br> entire text | 1-19 |
| A | JP 2021-103953 A (ABLE K.K.) 26 July 2021 (2021-07-26)<br> entire text | 1-19 |
| A | JP 2021-36804 A (HITACHI, LTD.) 11 March 2021 (2021-03-11)<br> entire text | 1-19 |

Form PCT/ISA/210 (second sheet) (January 2015)

37

**INTERNATIONAL SEARCH REPORT**
Information on patent family members

<table>
<tr><td colspan="2">International application No.</td></tr>
<tr><td colspan="2">**PCT/JP2023/004809**</td></tr>
</table>

| Patent document cited in search report | | | Publication date (day/month/year) | Patent family member(s) | Publication date (day/month/year) |
|---|---|---|---|---|---|
| WO | 2016/063364 | A1 | 28 April 2016 | US 2017/0306287 A1 claims, paragraphs [0030]-[0097], fig. 1-10 CN 107075437 A | |
| JP | 63-44150 | A | 25 February 1988 | (Family: none) | |
| JP | 2021-101153 | A | 08 July 2021 | (Family: none) | |
| JP | 61-808 | U1 | 07 January 1986 | (Family: none) | |
| WO | 2016/013395 | A1 | 28 January 2016 | US 2017/0191019 A1 the whole sentences | |
| JP | 2012-217426 | A | 12 November 2012 | (Family: none) | |
| JP | 2004-101196 | A | 02 April 2004 | (Family: none) | |
| JP | 2021-103953 | A | 26 July 2021 | (Family: none) | |
| JP | 2021-36804 | A | 11 March 2021 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014507959 A